(19) Europäisches Patentamt
European Patent Office
Office européen des brevets

(11) EP 2 123 301 A1

(12) EUROPEAN PATENT APPLICATION
published in accordance with Art. 153(4) EPC

(43) Date of publication:
**25.11.2009 Bulletin 2009/48**

(21) Application number: **07859850.5**

(22) Date of filing: **12.12.2007**

(51) Int Cl.:
***A61K 45/00*** *(2006.01)* ***A61K 31/4985*** *(2006.01)*
***A61K 31/519*** *(2006.01)* ***A61P 13/00*** *(2006.01)*
***A61P 13/02*** *(2006.01)* ***A61P 13/04*** *(2006.01)*
***A61P 43/00*** *(2006.01)* ***C07D 487/04*** *(2006.01)*
***C07D 495/04*** *(2006.01)*

(86) International application number:
**PCT/JP2007/074361**

(87) International publication number:
**WO 2008/072778 (19.06.2008 Gazette 2008/25)**

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **13.12.2006 JP 2006336217**

(71) Applicant: **ASKA Pharmaceutical Co., Ltd.**
**Minato-ku, Tokyo 108-8532 (JP)**

(72) Inventors:
- **GOTANDA, Kotaro**
**Kawasaki-shi**
**Kanagawa 213-8522 (JP)**
- **SHINBO, Atsushi**
**Kawasaki-shi**
**Kanagawa 213-8522 (JP)**
- **NAKANO, Youichi**
**Kawasaki-shi**
**Kanagawa 213-8522 (JP)**
- **KOBAYASHI, Hideo**
**Kawasaki-shi**
**Kanagawa 213-8522 (JP)**
- **OKADA, Makoto**
**Kawasaki-shi**
**Kanagawa 213-8522 (JP)**
- **ASAGARASU, Akira**
**Kawasaki-shi**
**Kanagawa 213-8522 (JP)**

(74) Representative: **Albrecht, Thomas**
**Kraus & Weisert**
**Patent- und Rechtsanwälte**
**Thomas-Wimmer-Ring 15**
**D-80539 München (DE)**

# (54) THERAPEUTIC AGENT FOR URINARY TRACT DISEASE

(57) Disclosed is a treating agent of overactive bladder syndrome, pollakiuria, urinary incontinence, dysuria in benign prostatic hyperplasia or urolithiasis, which comprises a compound having PDE9-inhibiting activity as the active ingredient.



Printed by Jouve, 75001 PARIS (FR)

## Description

### Technical Field

This invention relates to a treating agent of overactive bladder syndrome, pollakiuria, urinary incontinence, dysuria in benign prostatic hyperplasia or urolithiasis, which is characterized by comprising a compound having phosphodiesterase type 9 (PDE9)-inhibiting activity as the active ingredient.

### Background Art

Dysuria can be largely divided into emptying disorder due to inability to urinate with sufficient force at the time of emptying the bladder, and bladder-filling disorder due to inability to retain urine during the filling time. Presently, $\alpha_1$ blocker is frequently used for treating the emptying disorder and anticholine agent, for treating bladder-filling disorder. These drugs, however, have such defects as insufficient long-term therapeutic effect or reduction in quality of life (QOL) induced by side effect, and development of drugs having new activity mechanism different from the conventional approach, for example, drugs utilizing potassium channel opening activity, cyclic guanosine-3',5'-monophosphate (cGMP) degradation inhibiting activity, is in demand.

cGMP plays an important role in variegated cellular events such as smooth muscle relaxation, memory and learning function control, photoreaction of retina, cell proliferation, immunoreaction and the like. In normal cells, cGMP synthesis by nitrogen monoxide-(NO)-cGMP system and cGMP degradation by PDE system are maintained at balanced levels. Whereas, within the cells under various states of disorder, function of the NO-cGMP system lowers to render the cGMP synthesis level in the cells low, while the cGMP degradation level is unchanged. Hence, cGMP concentration in the affected cells becomes low. It is expected, therefore, prevention of cGMP degradation in the cells to redress the reduction in intracellular cGMP concentration would be useful for treating or preventing the diseases.

While there are many types of PDE, those which specifically degrade cGMP are type 5 (PDE5), type 6 (PDE6) and type 9 (PDE9). Of these, PDE9 shows the least Km value (J. Biol. Chemistry, Vol. 273, No. 25, 15559 - 15564 (1998)), has high affinity to cGMP and is considered to participate in degradation of cGMP with particular significance.

As the compounds having PDE9-inhibiting activity, pyrazolopyrimidine derivatives are known. Prior art references reported about the derivatives, for example, that they were useful for treating insulin-resistant diseases or the circulatory system disorder, and for improving perception, learning and memory functions (cf. PCT International Publications WO 03/037432 Pamphlet, WO 03/037899 Pamphlet and WO 2004/018474 Pamphlet).

However,there exists no literature discussing relevancy of PDE9-inhibiting activity to therapeutic efficacy on uropathy.

### Disclosure of the Invention

The object of the present invention is to offer a treating agent of uropathy, based on PDE9-inhibiting activity.

We have discovered that inhibition of PDE9 is effective for therapeutic treatment of various disorders of urinary tract such as overactive bladder syndrome, pollakiuria, urinary incontinence, dysuria in benign prostatic hyperplasia or urolithiasis, and come to complete the present invention.

Thus, according to the present invention, a treating agent of overactive bladder syndrome, pollakiuria, urinary incontinence, dysuria in benign prostatic hyperplasia or urolithiasis is provided, which is characterized by comprising a compound having PDE9-inhibiting activity as the active ingredient.

In this specification, "a compound having PDE9-inhibiting activity" refers to such a compound of which $IC_{50}$ value to PDE9 is 1 $\mu$mol/L or less, preferably 100 nmol/L or less. $IC_{50}$ value of the compound can be measured by, for example, the experiment shown under the later-appearing item, "Measurement of PDE9-inhibiting activity".

Any compounds of which $IC_{50}$ value to PDE9 is 1 $\mu$mol/L or less can be used as the active ingredient in the present invention without particular limitation. Whereas, as specific examples of the active ingredient, compounds of the following formulae (I)-(V) can be named:

thienopyrimidine derivatives represented by Formula (I),

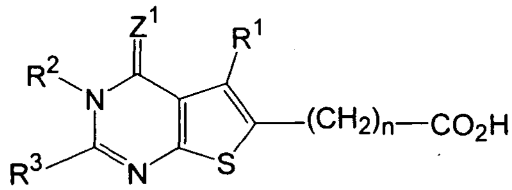

( I )

in the formula,
$R^1$ stands for hydrogen, $C_{1-6}$ alkyl, $C_{1-6}$ alkoxy$C_{1-6}$ alkyl, or $C_{1-6}$ haloalkyl having 1-9 halogen atoms,
$R^2$ stands for hydrogen, $C_{1-6}$ alkyl, phenyl$C_{1-6}$ alkyl or amino,
$R^3$ either stands for $C_{2-6}$ alkyl, $C_{2-6}$ alkenyl, carbamoyl$C_{1-6}$ alkyl, amino$C_{1-6}$ alkyl, $C_{1-6}$ alkylamino$C_{1-6}$ alkyl, di-($C_{1-6}$ alkyl) amino$C_{1-6}$ alkyl, $C_{1-6}$ alkylthio or Y-X-, or
$R^2$ and $R^3$ may together form tetramethylene,
X stands for a direct bond or $CH_2$, CH(OH), $CH(C_6H_5)$, CO, $CH_2CH_2$, $CH_2CO$, $COCH_2$, S, O, or NH,
Y stands for an aromatic carbocyclic group, aromatic heterocyclic group, $C_{4-7}$ cycloalkyl, $C_{4-7}$ cycloalkenyl, 5- to 7-membered saturated heterocyclic group containing 1 or 2 nitrogen atoms, or 5- to 7-membered saturated heterocyclic group forming a condensed ring with a 5- to 6-membered saturated cyclic group and containing 1 or 2 nitrogen atoms, each of which may be optionally substituted with 1-3 substituents selected from halogen, $C_{1-6}$ alkyl, $C_{1-6}$ haloalkyl having 1-9 halogen atoms, $C_{1-5}$ alkoxy, $C_{1-6}$ haloalkoxy having 1-9 halogen atoms, $C_{1-6}$ alkylthio, $C_{1-6}$ haloalkylthio having 1-9 halogen atoms, $C_{1-4}$ alkylenedioxy, carboxy, $C_{1-6}$ alkoxycarbonyl, oxo, amino, nitro and phenyl,
$Z^1$ stands for S or O, and
n is 0 or an integer of 1-4,
or salts thereof;
quinazoline derivatives represented by Formula (II),

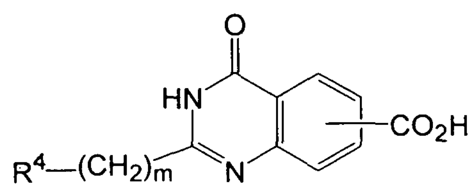

( II )

in the formula,
$R^4$ stands for phenyl or aromatic heterocyclic group which may be optionally substituted with 1-3 substituents selected from halogen, $C_{1-6}$ alkyl, $C_{1-6}$ haloalkyl having 1-9 halogen atoms and $C_{1-6}$ alkoxy, and
m is an integer of 1-3,
or salts thereof;
quinoxaline derivatives represented by Formula (III),

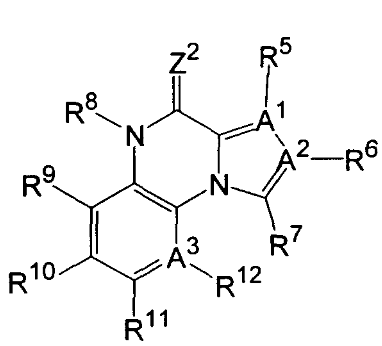

( III )

in the formula,
$R^5$ and $R^6$ each independently stands for hydrogen; halogen; $C_{1-6}$ alkyl or $C_{1-6}$ alkoxy each of which is optionally substituted with hydroxy, halogen, $C_{1-6}$ alkoxy, $C_{1-6}$ haloalkoxy having 1 - 9 halogen atoms, carboxy, $C_{1-6}$ alkoxycarbonyl, $C_{1-6}$ alkanoyl, amino, amido, carbamoyl, oxo, carbocyclic group or heterocyclic group; acyl which is optionally substituted with hydroxy, $C_{1-6}$ alkyl, $C_{2-6}$ alkenyl, $C_{2-6}$ alkynyl, $C_{1-6}$ haloalkyl having 1 - 9 halogen atoms, $C_{1-6}$ alkoxy, $C_{1-6}$ haloalkoxy having 1 - 9 halogen atoms, amino, carbocyclic group or heterocyclic group; amino which is optionally substituted with 1 - 2 substituents selected from $C_{1-6}$ alkyl, $C_{2-6}$ alkenyl, $C_{2-6}$ alkynyl, alkanoyl, carbocyclic group and heterocyclic group; hydroxy; or pyrimidinyl which is optionally substituted with halogen, $C_{1-6}$ alkyl, $C_{1-6}$ alkoxy, $C_{1-6}$ haloalkoxy having 1 - 9 halogen atoms, nitro or amino,
$R^7$ stands for $C_{1-9}$ alkyl, $C_{2-9}$ alkenyl or $C_{2-9}$ alkynyl which are optionally substituted with hydroxy, halogen, $C_{1-6}$ alkoxy, $C_{1-6}$ haloalkoxy having 1 - 9 halogen atoms, carboxy, $C_{1-6}$ alkoxycarbonyl, alkanoyl, amino (here the amino group may further be substituted with 1 - 2 substituents selected from $C_{1-6}$ alkyl, $C_{2-6}$ alkenyl, $C_{2-6}$ alkynyl, $C_{1-6}$ haloalkyl having 1 - 9 halogen atoms, alkanoyl, carbocyclic group and heterocyclic group), amido, carbamoyl, oxo, carbocyclic or heterocyclic group (here the carbocyclic group and heterocyclic group each may further be substituted with hydroxy, halogen, $C_{1-6}$ alkyl, $C_{2-6}$ alkenyl, $C_{2-6}$ alkynyl, $C_{1-6}$ alkoxy, carboxy, $C_{1-6}$ alkoxycarbonyl, amino, amido or carbamoyl); aryl, saturated carbocyclic group or saturated heterocyclic group, each of which is optionally substituted with halogen, hydroxy, $C_{1-6}$ alkyl, $C_{2-6}$ alkenyl, $C_{2-6}$ alkynyl, $C_{1-6}$ alkoxy (here the $C_{1-6}$ alkyl, $C_{2-6}$ alkenyl, $C_{2-6}$ alkynyl and$C_{1-6}$alkoxy may further be substituted with halogen, hydroxy, $C_{1-6}$ alkoxy, $C_{1-6}$haloalkoxy having 1 - 9 halogen atoms, carboxy, $C_{1-6}$ alkoxycarbonyl, alkanoyl, amino, amido, carbamoyl, carbocyclic group or heterocyclic group, independently of each other), $C_{1-6}$ haloalkoxy having 1 - 9 halogen atoms, carboxy, $C_{1-6}$ alkoxycarbonyl, alkanoyl, amino, amido, carbamoyl, carbocyclic group or heterocyclic group; carboxy; $C_{1-6}$ alkoxycarbonyl (here the $C_{1-6}$ alkoxy moiety in the $C_{1-6}$ alkoxycarbonyl may further be substituted with hydroxy, halogen, $C_{1-6}$ alkoxy, $C_{1-6}$ haloalkoxy having 1 - 9 halogen atoms, carboxy, $C_{1-6}$ alkoxycarbonyl, amino, amido, carbamoyl, carbocyclic group or heterocyclic group amido (here the amino moiety in the amido may further be substituted with 1 - 2 substituents selected from $C_{1-6}$ alkyl, $C_{2-6}$ alkenyl, $C_{2-6}$ alkynyl, $C_{1-6}$ haloalkyl having 1 - 9 halogen atoms, alkanoyl, carbocyclic group and heterocyclic group); or carbamoyl (here the amino moiety in the carbamoyl may further be substituted with 1 - 2 substituents selected from $C_{1-6}$ alkyl, $C_{2-6}$ alkenyl, $C_{2-6}$ alkynyl, $C_{1-6}$ haloalkyl having 1 - 9 halogen atoms, alkanoyl, carbocyclic group and heterocyclic group),
$R^8$ stands for hydrogen; hydroxy; $C_{1-6}$ alkyl which is optionally substituted with hydroxy, halogen, $C_{1-6}$ alkoxy, $C_{1-6}$ haloalkoxy having 1 - 9 halogen atoms, carboxy, $C_{1-6}$ alkoxycarbonyl, alkanoyl, amino, amido, carbamoyl or oxo; or amino which is optionally substituted with 1 - 2 substituents selected from $C_{1-6}$ alkyl, $C_{2-6}$ alkenyl, $C_{2-6}$ alkynyl, $C_{1-6}$ haloalkyl having 1 - 9 halogen atoms, alkanoyl, carbocyclic group and heterocyclic group,
$R^9$ and $R^{12}$ each independently stands for hydrogen; halogen; $C_{1-6}$ alkyl, $C_{2-6}$ alkenyl or $C_{1-6}$ alkoxy each of which is optionally substituted with hydroxy, halogen, $C_{1-6}$ alkoxy, $C_{1-6}$ haloalkoxy having 1 - 9 halogen atoms, carboxy, $C_{1-6}$ alkoxycarbonyl, alkanoyl, amino, amido, carbamoyl or oxo; cyano; or nitro,
$R^{10}$ and $R^{11}$ each independently stands for hydrogen; halogen; $C_{1-6}$ alkyl, $C_{2-6}$ alkenyl, $C_{2-6}$ alkynyl or $C_{1-6}$ alkoxy each of which is optionally substituted with hydroxy, halogen, $C_{1-6}$ alkoxy, $C_{1-6}$ haloalkoxy having 1 - 9 halogen atoms, carboxy, $C_{1-6}$ alkoxycarbonyl, alkanoyl, amino (here the amino may further be substituted with 1 - 2 substituents selected from $C_{1-6}$ alkyl, $C_{2-6}$ alkenyl, $C_{2-6}$ alkynyl, $C_{1-6}$ haloalkyl having 1 - 9 halogen atoms, alkanoyl, carbocyclic group and heterocyclic group), amido, carbamoyl, oxo, carbocyclic group or heterocyclic group (here the carbocyclic group and heterocyclic group each may further be substituted with hydroxy, halogen, $C_{1-6}$ alkyl, $C_{2-6}$ alkenyl, $C_{2-6}$ alkynyl, $C_{1-6}$ alkoxy, carboxy, $C_{1-6}$ alkoxycarbonyl, amino, amido or carbamoyl); cyano; amino which is optionally substituted with 1 - 2 substituents selected from $C_{1-6}$ alkyl, $C_{2-6}$ alkenyl, $C_{2-6}$ alkynyl (here the $C_{1-6}$ alkyl, $C_{2-6}$ alkenyl and $C_{2-6}$ alkynyl may further be substituted with, independently of each other, hydroxy, halogen, $C_{1-6}$ alkoxy, $C_{1-6}$ haloalkoxy having 1 - 9 halogen atoms, carboxy, $C_{1-6}$ alkoxycarbonyl, $C_{1-6}$ alkanoyl, amino, amido, carbamoyl, oxo, carbocyclic group and heterocyclic group), alkanoyl, carbocyclic group and heterocyclic group (here the carbocyclic group and heterocyclic group each may further be substituted with hydroxy, halogen, $C_{1-6}$ alkyl, $C_{2-6}$ alkenyl, $C_{2-6}$ alkynyl, $C_{1-6}$ alkoxy, carboxy, $C_{1-6}$ alkoxycarbonyl, amino, amido or carbamoyl); carbocyclic group or heterocyclic group each of which is optionally substituted with hydroxy, halogen, $C_{1-6}$ alkyl, $C_{2-6}$ alkenyl, $C_{2-6}$ alkynyl, $C_{1-6}$ alkoxy (here the $C_{1-6}$ alkyl, $C_{2-6}$ alkenyl, $C_{2-6}$ alkynyl and $C_{1-6}$ alkoxy may further be substituted with, independently of each other, hydroxy, halogen, $C_{1-6}$ alkoxy, $C_{1-6}$ haloalkoxy having 1 - 9 halogen atoms, carboxy, $C_{1-6}$ alkoxycarbonyl, $C_{1-6}$ alkanoyl, amino, amido, carbamoyl, oxo, carbocyclic group or heterocyclic group), $C_{1-6}$ haloalkoxy having 1- 9 halogen atoms, carboxy, $C_{1-6}$ alkoxycarbonyl, amino, amido or carbamoyl; $COR^{13}$; or $SO_2R^{13}$,
$R^{13}$ stands for hydrogen; hydroxy; $C_{1-6}$ alkyl, $C_{2-6}$ alkenyl, $C_{2-6}$ alkynyl or $C_{1-6}$ alkoxy, each of which is optionally substituted with hydroxy, halogen, $C_{1-6}$ alkoxy, $C_{1-6}$ haloalkoxy having 1 - 9 halogen atoms, carboxy, $C_{1-6}$ alkoxycarbonyl, alkanoyl, amino (here the amino may further be substituted with 1 - 2 substituents selected from $C_{1-6}$ alkyl, $C_{2-6}$ alkenyl, $C_{2-6}$ alkynyl, $C_{1-6}$ haloalkyl having 1 - 9 halogen atoms, alkanoyl, carbocyclic group and heterocyclic group), amido, oxo, carbocyclic group or heterocyclic group (here the carbocyclic group and heterocyclic group

each may further be substituted with hydroxy, halogen, $C_{1-6}$ alkyl, $C_{2-6}$ alkenyl, $C_{2-6}$ alkynyl, $C_{1-6}$ alkoxy, carboxy, $C_{1-6}$ alkoxycarbonyl, amino, amido or carbamoyl); amino which may be substituted with 1 - 2 substituents selected from $C_{1-6}$ alkyl, $C_{2-6}$ alkenyl, $C_{2-6}$ alkynyl, (here the $C_{1-6}$ alkyl, $C_{2-6}$ alkenyl and $C_{2-6}$ alkynyl may further be substituted with, independently of each other, hydroxy, halogen, $C_{1-6}$ alkoxy, $C_{1-6}$ haloalkoxy having 1 - 9 halogen atoms, carboxy, $C_{1-6}$ alkoxycarbonyl, alkanoyl, amino, amido, carbamoyl, oxo, carbocyclic group or heterocyclic group), $C_{1-6}$ haloalkyl having 1 - 9 halogen atoms, alkanoyl, carbocyclic group and heterocyclic group (here the carbocyclic group and heterocyclic group each may further be substituted with hydroxy, halogen, $C_{1-6}$ alkyl, $C_{2-6}$ alkenyl, $C_{2-6}$ alkynyl, $C_{1-6}$ alkoxy, carboxy, $C_{1-6}$ alkoxycarbonyl, amino, amido or carbamoyl or aziridin-1-yl, azetidin-1-yl, pyrrolidin-1-yl, piperidin-1-yl, piperazin-1-yl, morpholin-1-yl or pyrazol-1-yl, each of which may be substituted with 1 - 2 substituents selected from hydroxy, halogen, $C_{1-6}$ alkyl, $C_{2-6}$ alkenyl, $C_{2-6}$ alkynyl, $C_{1-6}$ alkoxy (here the $C_{1-6}$ alkyl, $C_{2-6}$ alkenyl, $C_{2-6}$ alkynyl and $C_{1-6}$ alkoxy may further be substituted with, independently of each other, hydroxy, halogen, $C_{1-6}$ alkoxy, $C_{1-6}$ haloalkoxy having 1 - 9 halogen atoms, carboxy, $C_{1-6}$ alkoxycarbonyl, alkanoyl, amino, amido, carbamoyl, oxo, carbocyclic group or heterocyclic group), $C_{1-6}$ haloalkoxy having 1 - 9 halogen atoms, carboxy, $C_{1-6}$ alkoxycarbonyl, alkanoyl, amino (here the amino may further be substituted with 1 - 2 substituents selected from $C_{1-6}$ alkyl, $C_{2-6}$ alkenyl, $C_{2-6}$ alkynyl, $C_{1-6}$ haloalkyl having 1 - 9 halogen atoms, alkanoyl, carbocyclic group and heterocyclic group), amido, carbamoyl, oxo, carbocyclic group and heterocyclic group (here the carbocyclic group and heterocyclic group each may further be substituted with hydroxy, halogen, $C_{1-6}$ alkyl, $C_{2-6}$ alkenyl, $C_{2-6}$ alkynyl, $C_{1-6}$ alkoxy, carboxy, $C_{1-6}$ alkoxycarbonyl, amino, amido or carbamoyl),

$Z^2$ stands for S or O,

$A^1$, $A^2$ and $A^3$ stand for N or C, independently of each other, with the proviso that $R^5$, $R^6$ and $R^{12}$ are respectively absent where $A^1$, $A^2$ and $A^3$ respectively stand for N,

or salts thereof;

pyrazolopyrimidine derivatives represented by Formula (IV),

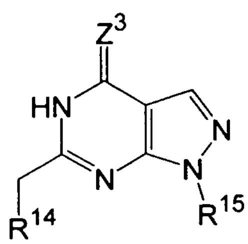

( IV )

in the formula,

$R^{14}$ stands for phenyl which is substituted with 1-5 substituents selected from halogen, $C_{1-6}$ alkyl, trifluoromethyl, trifluoromethoxy, cyano, hydroxy, nitro and $C_{1-6}$ alkoxy,

$R^{15}$ stands for pentan-3-yl or $C_{4-6}$ cycloalkyl, and

$Z^3$ stands for S or O,

or salts thereof; and

pyrazolopyrimidine derivatives represented by Formula (V)

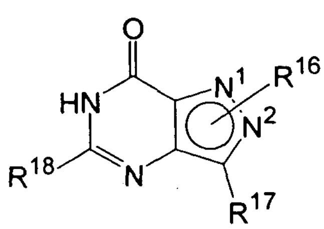

( V )

in the formula,

$R^{16}$ stands for hydrogen or $C_{1-6}$ alkyl, here the $R^{16}$ binding to $N^1$ or $N^2$,

$R^{17}$ stands for $C_{1-6}$ alkyl which is optionally substituted with hydroxy or alkoxy; $C_{3-7}$ cycloalkyl which is optionally substituted with alkyl, hydroxy or alkoxy; saturated 5- to 6-membered heterocyclic ring which is optionally substituted with alkyl, hydroxy or alkoxy; $het^1$; or $Ar^1$,

$R^{18}$ stands for $C_{1-6}$ alkyl which is optionally substituted with 1-2 substituents selected from optionally $Ar^2$-substituted

or $C_{1-6}$ alkyl-substituted $C_{3-7}$ cycloalkyl, $OAr^2$, $SAr^2$, $NHC(O)C_{1-6}$ alkyl, $het^2$, xanthine and naphthalene, here $Ar^1$ and $Ar^2$ standing for the group represented by the following formula (VI), independently of each other,

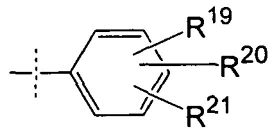

(VI)

[in the formula, $R^{19}$, $R^{20}$ and $R^{21}$ are either selected from hydrogen, halogen, phenoxy, phenyl, $CF_3$, $OCF_3$, $R^{22}$, $SR^{22}$ and $OR^{22}$ (here $R^{22}$ standing for $het^3$ or $C_{1-6}$ alkyl which is optionally substituted with phenyl, which phenyl being optionally further substituted with 1-3 substituents selected from halogen, $CF_3$, $OCF_3$, $C_{1-6}$ alkyl and $C_{1-6}$ alkoxy), or $R^{19}$ and $R^{20}$ together forming 3- or 4-atomic linker optionally containing 1-2 hetero atoms selected from O, S and N], here $het^1$, $het^2$ and $het^3$ may be the same or different, standing for aromatic 5- to 6-membered heterocyclic ring containing 1-3 hetero atoms selected from O, S and N, the heterocyclic ring being optionally substituted with 1-3 substituents selected from $C_{1-6}$ alkyl, $C_{1-6}$ alkoxy, halogen, and phenyl which may further be substituted with 1-3 substituents selected from halogen and $C_{1-6}$ alkyl, or salts thereof.

In the present specification, the expressions such as "$C_{16}$", "$C_{1-4}$", "$C_{1-9}$" "$C_{2-6}$", "$C_{2-9}$", "$C_{3-7}$", "$C_{3-8}$", "$C_{4-6}$", "$C_{4-7}$" and "$C_{5-7}$" signify that the carbon number of the group to which such a prefix is attached is within the given numerical range.

"$C_{1-6}$ alkyl" may be straight chain or branched, examples of which include methyl, ethyl, n-propyl, isopropyl, n-butyl, isobutyl, sec-butyl, tert-butyl, n-pentyl, n-hexyl and the like. Of these, methyl, ethyl, n-propyl, isopropyl and n-butyl are preferred. Also "$C_{1-9}$ alkyl" may be straight chain or branched, specific examples of which include, besides those exemplified as above $C_{1-6}$ alkyl groups, 1-ethyl-n-propyl, n-heptyl, n-octyl, n-nonyl, 2-ethyl-1,1-dimethyl-n-butyl, 1,2,3-trimethyl-n-butyl, 1,5-dimethyl-n-heptan-3-yl and the like. Of these, 1-ethyl-n-propyl is preferred. "$C_{2-6}$ alkyl" include those exemplified as to above $C_{1-6}$ alkyl excepting methyl, among which ethyl, n-propyl, isopropyl and n-butyl are particularly preferred.

"$C_{2-6}$ alkenyl" contains one or more double bond(s) at optional position(s) and may be straight chain or branched, specific examples including vinyl, allyl, isopropenyl, 1-butenyl, 2-butenyl, 3-butenyl, 1,3-butadienyl, 2-methylallyl, 1-pentenyl, 1-hexenyl and the like, among which vinyl, allyl and isopropenyl are preferred. Also "$C_{2-9}$ alkenyl" contains one or more double bond(s) at optional position(s) and may be straight chain or branched, specific examples including, besides those exemplified as to above $C_{2-6}$ alkenyl, 1-heptenyl, 1-octenyl, 1-nonenyl and the like.

"$C_{2-6}$ alkynyl" contains one or more triple bond(s) at optional position(s) and may be straight chain or branched, specific examples including ethynyl, propynyl, 1-pentynyl and the like, ethynyl being preferred among these. Also "$C_{2-9}$ alkynyl" contains one or more triple bond(s) at optional position(s) and may be straight chain or branched, specific examples including, besides those exemplified as to above $C_{2-6}$ alkynyl, 1-heptynyl, 4-ethylheptan-5-ynyl and the like.

"$C_{1-6}$ alkoxy" refers to oxy (O) group substituted with $C_{1-6}$ alkyl, examples of which include methoxy, ethoxy, n-propoxy, isopropoxy, n-butoxy, isobutyloxy, sec-butyloxy, tert-butoxy, n-pentyloxy, n-hexyloxy and the like. Of these, methoxy, ethoxy, n-propoxy, isopropoxy and n-butoxy are preferred.

"$C_{1-6}$ alkylthio" refers to thio (S) group substituted with $C_{1-6}$ alkyl, specific examples including methylthio, ethylthio, n-propylthio, isopropylthio, n-butylthio, isobutylthio, sec-butylthio, tert-butylthio, n-pentylthio, n-hexylthio and the like. Of these, methylthio, ethylthio, n-propylthio, isopropylthio and n-butylthio are preferred.

"$C_{1-6}$ alkanoyl" refers to carbonyl (C=O) group substituted with $C_{1-6}$ alkyl, specific examples including acetyl, propionyl, butyryl, pentanoyl, hexanoyl and the like, among which acetyl and propionyl are preferred.

"$C_{1-4}$ alkylenedioxy" includes, for example, methylenedioxy, ethylenedioxy, propylenedioxy, tetramethylenedioxy and the like, among which methylenedioxy and ethylenedioxy are preferred.

"$C_{3-8}$ cycloalkyl" includes, for example, cyclopropyl, cyclobutyl, cyclopentyl, cyclohexyl, cycloheptyl and cyclooctyl; and "$C_{4-7}$ cycloalkyl" includes cyclobutyl, cyclopentyl, cyclohexyl and cycloheptyl. Furthermore, "$C_{5-7}$ cycloalkyl" includes cyclopentyl, cyclohexyl and cycloheptyl. Among the respective examples, cyclopentyl and cyclohexyl are preferred.

Also "$C_{3-8}$ cycloalkenyl" includes cyclopropenyl, cyclobutenyl, cyclopentenyl, cyclohexenyl, cycloheptenyl and cyclooctenyl; "$C_{4-7}$ cycloalkenyl" includes cyclobutenyl, cyclopentenyl, cyclohexenyl and cycloheptenyl; and "$C_{5-7}$ cycloalkenyl" includes cyclopentenyl, cyclohexenyl and cycloheptenyl. Among the respective examples, cyclopentenyl and cyclohexenyl are preferred.

"Halogen" includes fluorine, chlorine, bromine and iodine, fluorine, chlorine and bromine atoms being particularly preferred.

"$C_{1-6}$ haloalkyl having 1 - 9 halogen atoms" means $C_{1-6}$ alkyl following the earlier given definition, which are substituted with 1 - 9 same or different halogen atoms, examples of which include fluoromethyl, trifluoromethyl, 1,2-dichloroethyl, 1-chloro-2-bromoethyl, pentabromoethyl, heptafluoropropyl, 1-chloro-n-propyl, 2-bromo-2-methylethyl, 3-chloro-n-pentyl, 2-bromo-3-chloro-n-hexyl and the like. Of those, $C_{1-2}$ alkyl substituted with 1 - 5 same or different halogen atoms are preferred.

Also "$C_{1-6}$ haloalkoxy having 1 - 9 halogen atoms" signifies oxy (O) group substituted with above "$C_{1-6}$ haloalkyl having 1 - 9 halogen atoms". Furthermore, "$C_{1-6}$ haloalkylthio having 1 - 9 halogen atoms" means thio (S) group substituted with above "$C_{1-6}$ haloalkyl having 1-6 halogen atoms".

The "$C_{1-6}$ alkoxy$C_{1-6}$ alkyl" in the definition of $R^1$ in the formula (I) means $C_{1-6}$ alkyl substituted with $C_{1-6}$ alkoxy as defined in the above, specific examples including methoxymethyl, methoxyethyl, methoxy-n-propyl, methoxy-n-butyl, methoxy-n-hexyl, ethoxymethyl, isopropoxymethyl, ethoxyethyl, n-butoxy-n-propyl and the like, among which methoxymethyl, methoxyethyl, ethoxymethyl and ethoxyethyl are preferred.

"Phenyl$C_{1-6}$ alkyl" in the definition of $R^2$ in the formula (I) signifies $C_{1-6}$ alkyl following the definition given in the above, which are substituted with phenyl; "carbamoyl$C_{1-6}$ alkyl" in the definition of $R^3$ in the formula (I) signifies $C_{1-6}$ alkyl following the definition given in the above, which is substituted with carbamoyl ($-CONH_2$); and "amino$C_{1-6}$ alkyl" signifies $C_{1-6}$ alkyl following the definition given in the above, which is substituted with amino ($-NH_2$).

"$C_{1-6}$ alkylamino$C_{1-6}$ alkyl" in the definition of $R^3$ in the formula (I) signifies the above amino$C_{1-6}$ alkyl in which the amino is further substituted with one of the $C_{1-6}$ alkyl following the definition given in the above; and "di-($C_{1-6}$ alkyl) amino$C_{1-6}$ alkyl" signifies those in which the amino is further substituted with two of the $C_{1-6}$ alkyl following the definition given in the above. Here the two $C_{1-6}$ alkyl groups substituting the amino in the di-($C_{1-6}$ alkyl)amino$C_{1-6}$ alkyl may be the same or different.

"$C_{1-6}$ alkoxycarbonyl" in the definition of Y in the formula (I) signifies carbonyl (CO) which is substituted with $C_{1-6}$ alkoxy following the definition given in the above.

"Aromatic carbocyclic group" in the definition of Y in the formula (I) and that of $R^7$ in the formula (III) includes $C_{6-20}$ aromatic carbocyclic groups, specific examples including phenyl, 1-indenyl, 1-naphthyl, 2-naphthyl, 2-anthryl, 1-acenaphthenyl and the like, among which phenyl, 1-naphthyl and 2-naphthyl are preferred.

"Aromatic heterocyclic group" in the definition of Y in the formula (I), that of $R^4$ in the formula (II) and that of $R^7$ in the formula (III) includes monocyclic or polycyclic aromatic heterocylic groups containing 1-2 hetero atoms selected from N, O and S, in which one ring is 5- to 6-membered ring, specific examples including pyrrolyl, furyl, thienyl, imidazolyl, pyrazolyl, oxazolyl, isoxazolyl, thiazolyl, pyridyl, pyrazinyl, pyrimidinyl, pyridazinyl, indolyl, benzimidazolyl, benzoxazolyl, benzthiazolyl, quinolyl, isoquinolyl, quinazolyl and the like, among which monocyclic aromatic heterocyclic groups are preferred.

As the "5- to 7-membered saturated heterocyclic group containing 1-2 nitrogen atoms" in the definition of Y in the formula (I), for example, pyrrolidinyl, piperidinyl, piperazinyl, azepinyl and the like can be named, among which piperidinyl and piperazinyl are preferred.

As the "5- to 7-membered saturated heterocyclic group forming a condensed ring with a 5- to 6-membered saturated cyclic group and containing 1 or 2 nitrogen atoms" in the definition of Y in the formula (I), for example, hexahydrocyclopenta[b]pyrrolyl, hexahydrocyclopenta[c]pyrrolyl, octahydrocyclopenta[b]pyridyl, octahydrocyclopenta [b]pyridyl, decahydrocyclopenta[b]azepinyl, octahydroindolyl, octahydroisoindolyl, decahydroquinolyl, decahydroisoquinolyl, dodecahydrobenzo[b]azepinyl, octahydropyrrolo[2,3-d]pyridyl, octahydropyrrolo[1,2-a]pyrazyl, octahydropyrido [1,2-a]pyrimidinyl, decahydrophthalazinyl, decahydronaphthyridinyl, decahydroquinazolinyl and the like can be named, among which decahydroquinolyl, decahydroisoquinolyl and octahydropyrrolo[1,2-a]pyrazyl are preferred.

The substitution site of the carboxy group on the benzene ring constituting the quinazoline skeleton in the formula (II) is not particularly limited, while 6- or 7-position of quinazoline is preferred, 7-position being particularly preferred.

"Saturated carbocyclic group" in the definition of $R^7$ in the formula (III) includes aforesaid $C_{3-8}$ cycloalkyl, among which cyclopentyl and cyclohexyl are preferred. Also "saturated heterocyclic group" in the definition of $R^7$ in the formula (III) means 5-to 7-membered saturated heterocyclic groups containing 1 - 3 hetero atoms selected from N, O and S, examples of which include pyrrolidinyl, furanyl, imidazolidinyl, pyrazolidinyl, oxathiolanyl, piperidinyl, piperazinyl, tetrahydropyranyl, morpholinyl, azepinyl, oxepinyl, diazepinyl and the like. Of these, pyrrolidinyl, piperidinyl and piperazinyl are preferred.

"Aryl" in the definition of $R^7$ in the formula (III) encompasses those groups named as examples of the aromatic carbocyclic group in the definition of Y in the formula (I) and those groups named as examples of the aromatic heterocyclic group in the definition of Y in the formula (I).

"Carbocyclic group" in the definitions of $R^5$-$R^8$, $R^{10}$, $R^{11}$ and $R^{13}$ in the formula (III) includes those aromatic carbocyclic groups and saturated carbocyclic groups previously defined. Also "heterocyclic group" in the definitions of $R^5$-$R^8$, $R^{10}$, $R^{11}$ and $R^{13}$ in the formula (III) includes the above-defined aromatic heterocyclic groups and saturated heterocyclic groups.

Furthermore, the compounds of the formula (IV) and those of the formula (V) are known, as described in, for example PCT International Publications WO 2004/018474 Pamphlet and WO 03/037899 Pamphlet, respectively, and these Publications are to be referred to concerning the definitions of terms such as the substituents in the compounds of the formulae (IV) and (V), production methods of the compounds of the formulae (IV) and (V), and so on.
A group of active ingredients preferred for the treating agent of the present invention are the compounds of the formula (I) in which $R^1$ stands for $C_{1-6}$ alkyl, in particular, methyl.
Another group of active ingredients preferred for the treating agent of the present invention are the compounds of the formula (I) in which $R^2$ stands for hydrogen.
A further group of active ingredients preferred for the treating agent of the present invention are the compounds of the formula (I) in which $R^3$ stands for Y-X- group, in particular, wherein X stands for $CH_2$, S, O or NH, inter alia, wherein X stands for $CH_2$.
Furthermore, among the compounds of the formula (I) in which $R^3$ stands for Y-X- group, those in which Y stands for aromatic carbocyclic group or aromatic heterocyclic group each of which is optionally substituted with 1-3 substituents selected from halogen, $C_{1-6}$ alkyl, $C_{1-6}$ haloalkyl containing 1-6 halogen atoms, $C_{1-6}$ alkoxy, $C_{1-6}$ haloalkoxy having 1-6 halogen atoms, $C_{1-6}$ alkylthio, $C_{1-6}$ haloalkylthio having 1-9 halogen atoms, $C_{1-4}$ alkylenedioxy, carboxy, $C_{1-6}$ alkoxycarbonyl, amino, nitro and phenyl, are particularly preferred.
Another preferred group of the active ingredients for the treating agent of the present invention are the compounds of the formula (I) in which $Z^1$ stands for O.
Still another preferred group of the active ingredients for the treating agent of the present invention are the compounds of the formula (I) in which n is 0.
A different group of preferred active ingredients for the treating agent of the present invention are the compounds of the formula (II) in which $R^4$ stands for phenyl which is optionally substituted with 1-3 substituents selected from halogen, $C_{1-6}$ alkyl, $C_{1-6}$ haloalkyl containing 1-6 halogen atoms and $C_{1-6}$ alkoxy.
A further different group of preferred active ingredients for the treating agent of the present invention are the compounds of the formula (II) in which m is 1.
A still different group of preferred active ingredients for the treating agent of the present invention are the compounds of the formula (III) in which $A^1$ stands for C and $R^5$ stands for hydrogen.
Another preferred group of preferred active ingredients for the treating agent of the present invention are the compounds of the formula (III) in which $A^2$ stands for N.
Another different group of preferred active ingredients for the treating agent of the present invention are the compounds of the formula (III) in which $A^3$ stands for C and $R^{12}$ stands for hydrogen.
A further different group of preferred active ingredients for the treating agent of the present invention are the compounds of the formula (III) in which $R^7$ stands for $C_{1-9}$ alkyl or $C_{2-9}$ alkenyl each of which is optionally substituted with hydroxy, halogen, $C_{1-6}$ alkoxy, $C_{1-6}$ haloalkoxy having 1 - 9 halogen atoms, carboxy, $C_{1-6}$ alkoxycarbonyl, alkanoyl, amino (here the amino may further be substituted with 1 - 2 substituents selected from $C_{1-6}$ alkyl, $C_{2-6}$ alkenyl, $C_{2-6}$ alkynyl, $C_{1-6}$ haloalkyl having 1 - 9 halogen atoms, alkanoyl, carbocyclic group and heterocyclic group), amido, carbamoyl, oxo, carbocyclic group or heterocyclic group (here the carbocyclic group and heterocyclic group each may further be substituted with hydroxy, halogen, $C_{1-6}$ alkyl, $C_{2-6}$ alkenyl, $C_{2-6}$ alkynyl, $C_{1-6}$ alkoxy, carboxy, $C_{1-6}$ alkoxycarbonyl, amino, amido or carbamoyl or saturated carbocyclic group which is optionally substituted with halogen, hydroxy, $C_{1-6}$ alkyl, $C_{2-6}$ alkenyl, $C_{2-6}$ alkynyl, $C_{1-6}$ alkoxy (here the $C_{1-6}$ alkyl, $C_{2-6}$ alkenyl, $C_{2-6}$ alkynyl and $C_{1-6}$ alkoxy may further be substituted with halogen, hydroxy, $C_{1-6}$ alkoxy, $C_{1-6}$ haloalkoxy having 1 - 9 halogen atoms, carboxy, $C_{1-6}$ alkoxycarbonyl, alkanoyl, amino, amido, carbamoyl, carbocyclic group or heterocyclic group, independently of each other), $C_{1-6}$ haloalkoxy having 1 - 9 halogen atoms, carboxy, $C_{1-6}$ alkoxycarbonyl, alkanoyl, amino, amido, carbamoyl, carbocyclic group or heterocyclic group. In particular, the compounds of the formula (III) in which $R^7$ stands for $C_{1-9}$ alkyl or $C_{2-9}$ alkenyl which may be substituted with carboxy; or $C_{5-7}$ cycloalkyl, are preferred.
Another group of preferred active ingredients for the treating agent of the present invention are the compounds of the formula (III) in which $R^8$ stands for hydrogen; or $C_{1-6}$ alkyl which is optionally substituted with hydroxy, halogen, $C_{1-6}$ alkoxy, $C_{1-6}$ haloalkoxy having 1-9 halogen atoms, carboxy, $C_{1-6}$ akoxycarbonyl, alkanoyl, amino, amido, carbamoyl or oxo. In particular, the compounds of the formula (III) in which $R^8$ stands for hydrogen or $C_{1-6}$ alkyl are preferred.
Still another different group of preferred active ingredients for the treating agent of the present invention are the compounds of the formula (III) in which $A^3$ stands for C and $R^9$ and $R^{12}$ both stand for hydrogen.
Another group of preferred active ingredients for the treating agent of the present invention are the compounds of the formula (III) in which $R^{10}$ is halogen; $C_{1-6}$ alkyl which is optionally substituted with hydroxy, halogen, $C_{1-6}$ alkoxy, $C_{1-6}$ haloalkoxy having 1 - 9 halogen atoms, carboxy, $C_{1-6}$ alkoxycarbonyl, alkanoyl, amino (here the amino may further be substituted with 1 - 2 substituents selected from $C_{1-6}$ alkyl, $C_{2-6}$ alkenyl, $C_{2-6}$ alkynyl, $C_{1-6}$ haloalkyl having 1 - 9 halogen atoms, alkanoyl, carbocyclic group and heterocyclic group), amido, carbamoyl, oxo, carbocyclic group or heterocyclic group (here the carbocyclic group and heterocyclic group may each be further substituted with hydroxy, halogen, $C_{1-6}$ alkyl, $C_{2-6}$ alkenyl, $C_{2-6}$ alkynyl, $C_{1-6}$ alkoxy, carboxy, $C_{1-6}$ alkoxycarbonyl, amino, amido or carbamoyl); or $COR^{13}$,

and $R^{13}$ stands for amino which may be substituted with 1 - 2 substituents selected from $C_{1-6}$ alkyl, $C_{2-6}$ alkenyl, $C_{2-6}$ alkynyl, $C_{1-6}$ alkoxy (here the $C_{1-6}$ alkyl, $C_{2-6}$ alkenyl, $C_{2-6}$ alkynyl and $C_{1-6}$ alkoxy may further be substituted with, independently of each other, hydroxy, halogen, $C_{1-6}$ alkoxy, $C_{1-6}$ haloalkoxy having 1 - 9 halogen atoms, carboxy, $C_{1-6}$ alkoxycarbonyl, alkanoyl, amino, amido, carbamoyl, oxo, carbocyclic group or heterocyclic group), $C_{1-6}$ haloalkyl having 1 - 9 halogen atoms, alkanoyl, carbocyclic group and heterocyclic group (here the carbocyclic group and heterocyclic group each may further be substituted with hydroxy, halogen, $C_{1-6}$ alkyl, $C_{2-6}$ alkenyl, $C_{2-6}$ alkynyl, $C_{1-6}$ alkoxy, carboxy, $C_{1-6}$ alkoxycarbonyl, amino, amido or carbamoyl or aziridin-1-yl, azetidin-1-yl, pyrrolidin-1-yl, piperidin-1-yl, piperazin-1-yl, morpholin-1-yl or pyrazol-1-yl, each of which may be substituted with 1 - 2 substituents selected from hydroxy, halogen, $C_{1-6}$ alkyl, $C_{2-6}$ alkenyl, $C_{2-6}$ alkynyl, $C_{1-6}$ alkoxy (here the $C_{1-6}$ alkyl, $C_{2-6}$ alkenyl, $C_{2-6}$ alkynyl and $C_{1-6}$ alkoxy may further be substituted with, independently of each other, hydroxy, halogen, $C_{1-6}$ alkoxy, $C_{1-6}$ haloalkoxy having 1 - 9 halogen atoms, carboxy, $C_{1-6}$ alkoxycarbonyl, alkanoyl, amino, amido, carbamoyl, oxo, carbocyclic group or heterocyclic group), $C_{1-6}$ haloalkoxy having 1 - 9 halogen atoms, carboxy, $C_{1-6}$ alkoxycarbonyl, alkanoyl, amino (here the amino may further be substituted with 1 - 2 substituents selected from $C_{1-6}$ alkyl, $C_{2-6}$ alkenyl, $C_{2-6}$ alkynyl, $C_{1-6}$ haloalkyl having 1 - 9 halogen atoms, alkanoyl, carbocyclic group and heterocyclic group), amido, carbamoyl, oxo, carbocyclic group and heterocyclic group (here the carbocyclic group and heterocyclic group each may further be substituted with hydroxy, halogen, $C_{1-6}$ alkyl, $C_{2-6}$ alkenyl, $C_{2-6}$ alkynyl, $C_{1-6}$ alkoxy, carboxy, $C_{1-6}$ alkoxycarbonyl, amino, amido or carbamoyl). In particular, the compounds of the formula (III) in which $R^{10}$ stands for halogen; $C_{1-6}$ alkyl which may be substituted with halogen; or $COR^{13}$, and $R^{13}$ stands for amino which may be substituted with 1 or 2 $C_{1-6}$ alkyl group(s) or piperazin-1-yl which may be substituted with $C_{1-6}$ alkyl (here the $C_{1-6}$ alkyl may further be substituted with hydroxy) are preferred.

A further different group of preferred active ingredients for the treating agent of the present invention are the compounds of the formula (III) in which $R^{11}$ is halogen; or $C_{1-6}$ alkoxy which is optionally substituted with hydroxy, halogen, $C_{1-6}$ alkoxy, $C_{1-6}$ haloalkoxy having 1 - 9 halogen atoms, carboxy, $C_{1-6}$ alkoxycarbonyl, alkanoyl, amino (here the amino may further be substituted with 1 - 2 substituents selected from $C_{1-6}$ alkyl, $C_{2-6}$ alkenyl, $C_{2-6}$ alkynyl, $C_{1-6}$ haloalkyl having 1 - 9 halogen atoms, alkanoyl, carbocyclic group and heterocyclic group), amido, carbamoyl, oxo, carbocyclic group or heterocyclic group (here the carbocyclic group and heterocyclic group each may further be substituted with hydroxy, halogen, $C_{1-6}$alkyl, $C_{2-6}$ alkenyl, $C_{2-6}$ alkynyl, $C_{1-6}$ alkoxy, carboxy, $C_{1-6}$ alkoxycarbonyl, amino, amido or carbamoyl). Of these, the compounds of the formula (III) in which $R^{11}$ stands for halogen or $C_{1-6}$ alkoxy are particularly preferred.

Still another preferred group of the active ingredients for the treating agent of the present invention are those of the formula (III) in which $Z^2$ stands for O.

As specific examples of the compounds which are preferred as the active ingredients for the treating agent of the present invention, the following can be named:

5-methyl-4-oxo-2-(thiophen-3-ylmethyl)-3,4-dihydrothieno-[2,3-d]pyrimidine-6-carboxylic acid,
5-methyl-4-oxo-2-(thiophen-2-ylmethyl)-3,4-dihydrothieno-[2,3-d]pyrimidine-6-carboxylic acid,
2-(5-chlorothiophen-2-ylmethyl)-5-methyl-4-oxo-3,4-dihydrothieno[2,3-d]pyrimidine-6-carboxylic acid,
5-methyl-2-(4-methylthiazol-2-ylmethyl)-4-oxo-3,4-dihydrothieno[2,3-d]pyrimidine-6-carboxylic acid,
5-methyl-4-oxo-2-(pyridin-3-ylmethyl)-3,4-dihydrothieno-[2,3-d]pyrimidine-6-carboxylic acid,
2-(2-fluorobenzyl)-5-methyl-4-oxo-3,4-dihydrothieno-[2,3-d]pyrimidine-6-carboxylic acid,
2-(3-fluorobenzyl)-5-methyl-4-oxo-3,4-dihydrothieno-[2,3-d]pyrimidine-6-carboxylic acid,
2-(4-fluorobenzyl)-5-methyl-4-oxo-3,4-dihydrothieno-[2,3-d]pyrimidine-6-carboxylic acid,
2-(2-chlorobenzyl)-5-methyl-4-oxo-3,4-dihydrothieno-[2,3-d]pyrimidine-6-carboxylic acid,
2-(3-chlorobenzyl)-5-methyl-4-oxo-3,4-dihydrothieno-[2,3-d]pyrimidine-6-carboxylic acid, sodium salt thereof and the sodium salt •1/2 ethanolate,
2-(4-chlorobenzyl)-5-methyl-4-oxo-3,4-dihydrothieno-[2,3-d]pyrimidine-6-carboxylic acid,
2-(3-bromobenzyl)-5-methyl-4-oxo-3,4-dihydrothieno-[2,3-d]pyrimidine-6-carboxylic acid,
2-(4-bromobenzyl)-5-methyl-4-oxo-3,4-dihydrothieno-[2,3-d]pyrimidine-6-carboxylic acid,
5-methyl-2-(2-methylbenzyl)-4-oxo-3,4-dihydrothieno-[2,3-d]pyrimidine-6-carboxylic acid,
5-methyl-2-(3-methylbenzyl)-4-oxo-3,4-dihydrothieno-[2,3-d]pyrimidine-6-carboxylic acid,
5-methyl-2-(4-methylbenzyl)-4-oxo-3,4-dihydrothieno-[2,3-d]pyrimidine-6-carboxylic acid,
2-(2,6-dimethylbenzyl)-5-methyl-4-oxo-3,4-dihydrothieno-[2,3-d]pyrimidine-6-carboxylic acid,
2-(4-ethylbenzyl)-5-methyl-4-oxo-3,4-dihydrothieno-[2,3-d]pyrimidine-6-carboxylic acid,
2-(4-isopropylbenzyl)-5-methyl-4-oxo-3,4-dihydrothieno-[2,3-d]pyrimidine-6-carboxylic acid,
2-(4-tert-butylbenzyl)-5-methyl-4-oxo-3,4-dihydrothieno-[2,3-d]pyrimidine-6-carboxylic acid,
5-methyl-4-oxo-2-(2-trifluoromethylbenzyl)-3,4-dihydrothieno-[2,3-d]pyrimidine-6-carboxylic acid,
5-methyl-4-oxo-2-(3-trifluoromethylbenzyl)-3,4-dihydrothieno-[2,3-d]pyrimidine-6-carboxylic acid,
5-methyl-4-oxo-2-(4-trifluromethylbenzyl)-3,4-dihydrothieno-[2,3-d]pyrimidine-6-carboxylic acid,

2-(4-hydroxybenzyl)-5-methyl-4-oxo-3,4-dihydrothieno-[2,3-d]pyrimidine-6-carboxylic acid,
2-(2-methoxybenzyl)-5-methyl-4-oxo-3,4-dihydrothieno-[2,3-d]pyrimidine-6-carboxylic acid,
2-(3-methoxybenzyl)-5-methyl-4-oxo-3,4-dihydrothieno-[2,3-d]pyrimidine-6-carboxylic acid,
5-methyl-4-oxo-2-(2-trifluoromethoxybenzyl)-3,4-dihydrothieno[2,3-d]pyrimidine-6-carboxylic acid,
5-methyl-4-oxo-2-(3-trifluoromethoxybenzyl)-3,4-dihydrothieno[2,3-d]pyrimidine-6-carboxylic acid,
5-methyl-4-oxo-2-(4-trifluoromethoxybenzyl)-3,4-dihydrothieno[2,3-d]pyrimidine-6-carboxylic acid,
2-(2-benzyloxybenzyl)-5-methyl-4-oxo-3,4-dihydrothieno-[2,3-d]pyrimidine-6-carboxylic acid,
2-(3-benzyloxybenzyl)-5-methyl-4-oxo-3,4-dihydrothieno-[2,3-d]pyrimidine-6-carboxylic acid,
2-(2-ethoxybenzyl)-5-methyl-4-oxo-3,4-dihydrothieno-[2,3-d]pyrimidine-6-carboxylic acid,
2-(4-butoxybenzyl)-5-methyl-4-oxo-3,4-dihydrothieno-[2,3-d]pyrimidine-6-carboxylic acid,
2-(2,3-dichlorobenzyl)-5-methyl-4-oxo-3,4-dihydrothieno-[2,3-d]pyrimidine-6-carboxylic acid,
2-(2,4-dichlorobenzyl)-5-methyl-4-oxo-3,4-dihydrothieno-[2,3-d]pyrimidine-6-carboxylic acid,
2-(3,4-dichlorobenzyl)-5-methyl-4-oxo-3,4-dihydrothieno-[2,3-d]pyrimidine-6-carboxylic acid, sodium salt thereof and the sodium salt•1/2 ethanolate,
2-(3,4-dimethoxybenzyl)-5-methyl-4-oxo-3,4-dihydrothieno-[2,3-d]pyrimidine-6-carboxylic acid,
5-methyl-2-(3,4-methylenedioxybenzyl)-4-oxo-3,4-dihydrothieno[2,3-d]pyrimidine-6-carboxylic acid,
5-methyl-2-(4-methylthiobenzyl)-4-oxo-3,4-dihydrothieno-[2,3-d]pyrimidine-6-carboxylic acid,
5-methyl-4-oxo-2-(2-trifluoromethylbenzyl)-3,4-dihydrothieno-[2,3-d]pyrimidine-6-carboxylic acid,
2-(2-carboxybenzyl)-5-methyl-4-oxo-3,4-dihydrothieno-[2,3-d]pyrimidine-6-carboxylic acid,
2-(biphenyl-4-ylmethyl)-5-methyl-4-oxo-3,4-dihydrothieno-[2,3-d]pyrimidine-6-carboxylic acid,
2-(3-bromo-4-methoxybenzyl)-5-methyl-4-oxo-3, 4-dihydrothieno[2,3-d]pyrimidine-6-carboxylic acid,
2-(5-bromo-2-methoxybenzyl)-5-methyl-4-oxo-3,4-dihydrothieno[2,3-d]pyrimidine-6-carboxylic acid,
2-(2-chloro-5-trifluoromethylbenzyl)-5-methyl-4-oxo-3,4-dihydrothieno[2,3-d]pyrimidine-6-carboxylic acid,
2-(2-fluoro-3-trifluoromethylbenzyl)-5-methyl-4-oxo-3, 4-dihydrothieno[2,3-d]pyrimidine-6-carboxylic acid,
2-(2-fluoro-5-trifluoromethylbenzyl)-5-methyl-4-oxo-3,4-dihydrothieno[2,3-d]pyrimidine-6-carboxylic acid,
2-(3-fluoro-5-trifluoromethylbenzyl)-5-methyl-4-oxo-3,4-dihydrothieno[2,3-d]pyrimidine-6-carboxylic acid,
2-(4-fluoro-3-trifluoromethylbenzyl)-5-methyl-4-oxo-3,4-dihydrothieno[2,3-d]pyrimidine-6-carboxylic acid,
2-(5-fluoro-2-trifluoromethylbenzyl)-5-methyl-4-oxo-3,4-dihydrothieno[2,3-d]pyrimidine-6-carboxylic acid,
2-(3-chloro-2-fluorobenzyl)-5-methyl-4-oxo-3,4-dihydrothieno-[2,3-d]pyrimidine-6-carboxylic acid,
2-(3-chloro-4-fluorobenzyl)-5-methyl-4-oxo-3,4-dihydrothieno-[2,3-d]pyrimidine-6-carboxylic acid,
2-(5-chloro-2-fluorobenzyl)-5-methyl-4-oxo-3,4-dihydrothieno-[2,3-d]pyrimidine-6-carboxylic acid,
2-(2-chloro-6-fluorobenzyl)-5-methyl-4-oxo-3,4-dihydrothieno-[2,3-d]pyrimidine-6-carboxylic acid,
2-{3,5-bis(trifluoromethyl)benzyl}-5-methyl-4-oxo-3,4-dihydrothieno[2,3-d]pyrimidine-6-carboxylic acid,
2-(3,4-difluorobenzyl)-5-methyl-4-oxo-3,4-dihydrothieno-[2,3-d]pyrimidine-6-carboxylic acid,
2-(2,5-difluorobenzyl)-5-methyl-4-oxo-3,4-dihydrothieno-[2,3-d]pyrimidine-6-carboxylic acid,
2-(2,4-difluorobenzyl)-5-methyl-4-oxo-3,4-dihydrothieno-[2,3-d]pyrimidine-6-carboxylic acid,
2-(2,6-difluorobenzyl)-5-methyl-4-oxo-3,4-dihydrothieno-[2,3-d]pyrimidine-6-carboxylic acid,
5-methyl-4-oxo-2-(2,3,5-trifluorobenzyl)-3,4-dihydrothieno-[2,3-d]pyrimidine-6-carboxylic acid,
5-methyl-2-(naphthalene-1-ylmethyl)-4-oxo-3,4-dihydrothieno-[2,3-d]pyrimidine-6-carboxylic acid,
2-($\alpha$-hydroxybenzyl)-5-methyl-4-oxo-3,4-dihydrothieno-[2,3-d]pyrimidine-6-carboxylic acid,
2-benzhydryl-5-methyl-4-oxo-3,4-dihydrothieno[2,3-d]-pyrimidine-6-carboxylic acid,
5-methyl-4-oxo-2-(pyridin-2-ylmethyl)-3,4-dihydrothieno-[2,3-d]pyrimidine-6-carboxylic acid,
2-(cyclopent-1-enylmethyl)-5-methyl-4-oxo-3,4-dihydrothieno-[2,3-d]pyrimidine-6-carboxylic acid,
2-(cyclohex-1-enylmethyl)-5-methyl-4-oxo-3,4-dihydrothieno-[2,3-d]pyrimidine-6-carboxylic acid,
2-cyclopentylmethyl-5-methyl-4-oxo-3,4-dihydrothieno-[2,3-d]pyrimidine-6-carboxylic acid,
2-cyclohexylmethyl-5-methyl-4-oxo-3,4-dihydrothieno-[2,3-d]pyrimidine-6-carboxylic acid,
5-methyl-4-oxo-2-piperidinomethyl-3,4-dihydrothieno-[2,3-d]pyrimidine-6-carboxylic acid,
5-methyl-4-oxo-2-(4-oxopiperidinomethyl)-3,4-dihydrothieno-[2,3-d]pyrimidine-6-carboxylic acid,
2-(4-carboxypiperidinomethyl)-5-methyl-4-oxo-3,4-dihydrothieno[2,3-d]pyrimidine-6-carboxylic acid,
5-methyl-2-(1-decahydroquinolylmethyl)-4-oxo-3,4-dihydrothieno[2,3-d]pyrimidine-6-carboxylic acid,
2-(4-tert-butoxycarbonylpiperazin-1-ylmethyl)-5-methyl-4-oxo-3,4-dihydrothieno[2,3-d]pyrimidine-6-carboxylic acid, and dihydrochloride thereof,
2-(octahydropyrrolo[1,2-a]pyrazin-2-ylmethyl)-5-methyl-4-oxo-3,4-dihydrothieno[2,3-d]pyrimidine-6-carboxylic acid,
5-methyl-4-oxo-2-phenyl-3,4-dihydrothieno[2,3-d]pyrimidine-6-carboxylic acid,
5-methyl-2-(2-naphthyl)-4-oxo-3,4-dihydrothieno[2,3-d]-pyrimidine-6-carboxylic acid,
5-methyl-4-oxo-2-(2-pyridyl)-3,4-dihydrothieno[2,3-d]-pyrimidine-6-carboxylic acid,
5-methyl-4-oxo-2-(3-pyridyl)-3,4-dihydrothieno[2,3-d]-pyrimidine-6-carboxylic acid,

5-methyl-4-oxo-2-(pyrazin-2-yl)-3,4-dihydrothieno[2,3-d]-pyrimidine-6-carboxylic acid,
2-(2-furyl)-5-methyl-4-oxo-3,4-dihydrothieno[2,3-d]pyrimidine-6-carboxylic acid,
5-methyl-4-oxo-2-(thiophen-3-yl)-3,4-dihydrothieno[2,3-d]-pyrimidine-6-carboxylic acid,
5-methyl-4-oxo-2-phenethyl-3,4-dihydrothieno[2,3-d]-pyrimidine-6-carboxylic acid,
5-methyl-4-oxo-2-(β-oxophenethyl)-3,4-dihydrothieno[2,3-d]-pyrimidine-6-carboxylic acid,
2-[2-(3-chlorophenyl)-2-oxoethyl]-5-methyl-4-oxo-3,4-dihydrothieno[2,3-d]pyrimidine-6-carboxylic acid,
2-butyl-5-methyl-4-oxo-3,4-dihydrothieno[2,3-d]pyrimidine-6-carboxylic acid,
2-allyl-5-methyl-4-oxo-3,4-dihydrothieno[2,3-d]pyrimidine-6-carboxylic acid,
5-methyl-2-methylthio-4-oxo-3,4-dihydrothieno[2,3-d]-pyrimidine-6-carboxylic acid,
2-carbamoylmethyl-5-methyl-4-oxo-3,4-dihydrothieno[2,3-d]-pyrimidine-6-carboxylic acid,
2-(2-aminoethyl)-5-methyl-4-oxo-3,4-dihydrothieno[2,3-d]-pyrimidine-6-carboxylic acid hydrobromide,
5-methyl-4-oxo-2-phenoxy-3,4-dihydrothieno[2,3-d]pyrimidine-6-carboxylic acid,
5-methyl-4-oxo-2-phenylthio-3,4-dihydrothieno[2,3-d]-pyrimidine-6-carboxylic acid,
5-methyl-4-oxo-2-phenylamino-3,4-dihydrothieno[2,3-d]-pyrimidine-6-carboxylic acid,
3-benzyl-2,5-dimethyl-4-oxo-3,4-dihydrothieno[2,3-d]-pyrimidine-6-carboxylic acid,
2-(3,4-dichlorobenzyl)-3,5-dimethyl-4-oxo-3,4-dihydrothieno-[2,3-d]pyrimidine-6-carboxylic acid,
3-methyl-4-oxo-6,7,8,9-tetrahydro-4H-pyrido[1,2-a]thieno-[2,3-d]pyrimidine-2-carboxylic acid,
2-(3,4-dichlorobenzyl)-5-methyl-4-oxo-3,4-dihydrothieno[2,3-d]-pyrimidine-6-acetic acid,
2-(3,4-dichlorobenzyl)-5-methyl-4-oxo-3,4-dihydrothieno[2,3-d]-pyrimidine-6-propionic acid,
2-(3,4-dichlorobenzyl)-5-methyl-4-oxo-3,4-dihydrothieno[2,3-d]-pyrimidine-6-butyric acid,
2-(3,4-dichlorobenzyl)-4-oxo-5-trifluoromethyl-3,4-dihydrothieno[2,3-d]pyrimidine-6-carboxylic acid,
2-(3,4-dichlorobenzyl)-5-methoxymethyl-4-oxo-3,4-dihydrothieno[2,3-d]pyrimidine-6-carboxylic acid,
4-oxo-2-(thiophen-3-ylmethyl)-3,4-dihydrothieno[2,3-d]-pyrimidine-6-carboxylic acid,
4-oxo-2-(thiophen-2-ylmethyl)-3,4-dihydrothieno[2,3-d]-pyrimidine-6-carboxylic acid,
2-benzyl-4-oxo-3,4-dihydrothieno[2,3-d]pyrimidine-6-carboxylic acid,
2-(3-chlorobenzyl)-4-oxo-3,4-dihydrothieno[2,3-d]pyrimidine-6-carboxylic acid,
4-oxo-2-(3-trifluoromethylbenzyl)-3,4-dihydrothieno[2,3-d]-pyrimidine-6-carboxylic acid,
2-(3,4-dichlorobenzyl)-4-oxo-3,4-dihydrothieno[2,3-d]-pyrimidine-6-carboxylic acid,
2-(cyclopent-1-enylmethyl)-4-oxo-3,4-dihydrothieno[2,3-d]-pyrimidine-6-carboxylic acid,
5-methyl-2-(thiophen-3-ylmethyl)-4-thioxo-3,4-dihydrothieno-[2,3-d]pyrimidine-6-carboxylic acid,
5-methyl-2-(thiophen-2-ylmethyl)-4-thioxo-3,4-dihydrothieno-[2,3-d]pyrimidine-6-carboxylic acid,
2-benzyl-5-methyl-4-thioxo-3,4-dihydrothieno[2,3-d]-pyrimidine-6-carboxylic acid,
2-(3-bromobenzyl)-5-methyl-4-thioxo-3,4-dihydrothieno[2,3-d]-pyrimidine-6-carboxylic acid,
2-(3-chlorobenzyl)-5-methyl-4-thioxo-3,4-dihydrothieno[2,3-d]-pyrimidine-6-carboxylic acid,
5-methyl-4-thioxo-2-(3-trifluoromethylbenzyl)-3,4-dihydrothieno[2,3-d]pyrimidine-6-carboxylic acid,
2-(3,4-dichlorobenzyl)-5-methyl-4-thioxo-3,4-dihydrothieno-[2,3-d]pyrimidine-6-carboxylic acid,
2-(3-chloro-4-fluorobenzyl)-5-methyl-4-thioxo-3,4-dihydrothieno[2,3-d]pyrimidine-6-carboxylic acid,
2-(cyclohex-1-enylmethyl)-5-methyl-4-thioxo-3,4-dihydrothieno[2,3-d]pyrimidine-6-carboxylic acid,
2-(cyclopent-1-enylmethyl)-5-methyl-4-thioxo-3,4-dihydrothieno[2,3-d]pyrimidine-6-carboxylic acid, and 2-cyclopentylidenemethyl-5-methyl-4-thioxo-3,4-dihydrothieno-[2,3-d]pyrimidine-6-carboxylic acid,
2-benzyl-4-thioxo-3,4-dihydrothieno[2,3-d]pyrimidine-6-carboxylic acid,
2-(3,4-dichlorobenzyl)-4-thioxo-3,4-dihydrothieno[2,3-d]-pyrimidine-6-carboxylic acid,
2-benzyl-4-oxo-3,4-dihydrothieno[2,3-d]pyrimidine-6-carboxylic acid,
2-(5-chlorothiophen-2-ylmethyl)-4-oxo-3,4-dihydrothieno-[2,3-d]pyrimidine-6-carboxylic acid,
2-(2-fluorobenzyl)-4-oxo-3,4-dihydrothieno[2,3-d]pyrimidine-6-carboxylic acid,
2-(3-fluorobenzyl)-4-oxo-3,4-dihydrothieno[2,3-d]pyrimidine-6-carboxylic acid,
2-(4-nuorobenzyl)-4-oxo-3,4-dihydrothieno[2,3-d]pyrimidine-6- carboxylic acid,
2-(2-chlorobenzyl)-4-oxo-3,4-dihydrothieno[2,3-d]pyrimidine-6-carboxylic acid,
2-(4-chlorobenzyl)-4-oxo-3,4-dihydrothieno[2,3-d]pyrimidine-6-carboxylic acid,
2-(3-bromobenzyl)-4-oxo-3,4-dihydrothieno[2,3-d]pyrimidine-6-carboxylic acid,
2-(3-methylbenzyl)-4-oxo-3,4-dihydrothieno[2,3-d]pyrimidine-6-carboxylic acid,
4-oxo-2-(2-trifluoromethylbenzyl)-3,4-dihydrothieno[2,3-d]-pyrimidine-6-carboxylic acid,
2-(cyclohexen-1-ylmethyl)-4-oxo-3,4-dihydrothieno[2,3-d]-pyrimidine-6-carboxylic acid,
5-methyl-4-oxo-2-(thiophen-2-yl)-3,4-dihydrothieno[2,3-d]-pyrimidine-6-carboxylic acid,
2-(a-hydroxythiophen-2-ylmethyl)-5-methyl-4-oxo-3,4-dihydrothieno [2, 3-d]pyrimidine-6-carboxylic acid,
5-methyl-4-oxo-2- [(2-thiophen-2-yl)ethyl]-3,4-dihydrothieno-[2,3-d]pyrimidine-6-carboxylic acid,
5-methyl-4-oxo-2-(thiophen-2-ylcarbonyl)-3,4-dihydrothieno-[2,3-d]pyrimidine-6-carboxylic acid,
5-methyl-4-oxo-2-(thiophen-2-ylsulfanyl)-3,4-dihydrothieno-[2,3-d]pyrimidine-6-carboxylic acid,

5-methyl-4-oxo-2-(thiophen-2-yloxy)-3,4-dihydrothieno[2,3-d]-pyrimidine-6-carboxylic acid,
5-methyl-4-oxo-2-(thiophen-2-ylamino)-3,4-dihydrothieno-[2,3-d]pyrimidine-6-carboxylic acid,
2-(5-fluorothiophen-2-ylmethyl)-5-methyl-4-oxo-3,4-dihydrothieno[2,3-d]pyrimidine-6-carboxylic acid,
2-(5-bromothiophen-2-ylmethyl)-5-methyl-4-oxo-3,4-dihydrothieno[2,3-d]pyrimidine-6-carboxylic acid,
5-methyl-2-(5-methylthiophen-2-ylmethyl)-4-oxo-3,4-dihydrothieno[2,3-d]pyrimidine-6-carboxylic acid,
2-(5-fluorothiophen-3-ylmethyl)-5-methyl-4-oxo-3, 4-dihydrothieno[2,3-d]pyrimidine-6-carboxylic acid,
2-(5-chlorothiophen-3-ylmethyl)-5-methyl-4-oxo-3,4-dihydrothieno[2,3-d]pyrimidine-6-carboxylic acid,
2-(5-bromothiophen-3-ylmethyl)-5-methyl-4-oxo-3,4-dihydrothieno[2,3-d]pyrimidine-6-carboxylic acid,
5-methyl-2-(5-methylthiophen-3-ylmethyl)-4-oxo-3,4-dihydrothieno[2,3-d]pyrimidine-6-carboxylic acid,
2-(furan-2-ylmethyl)-5-methyl-4-oxo-3,4-dihydrothieno[2,3-d]-pyrimidine-6-carboxylic acid,
2-(furan-3-ylmethyl)-5-methyl-4-oxo-3,4-dihydrothieno[2,3-d]-pyrimidine-6-carboxylic acid,
2-(5-chlorofuran-2-ylmethyl)-5-methyl-4-oxo-3,4-dihydrothieno[2,3-d]pyrimidine-6-carboxylic acid,
2-(5-chlorofuran-3-ylmethyl)-5-methyl-4-oxo-3,4-dihydrothieno[2,3-d]pyrimidine-6-carboxylic acid,
2-(5-chlorooxazol-2-ylmethyl)-5-methyl-4-oxo-3,4-dihydrothieno[2,3-d]pyrimidine-6-carboxylic acid,
5-methyl-4-oxo-2-(pyridin-4-ylmethyl)-3,4-dihydrothieno-[2,3-d]pyrimidine-6-carboxylic acid,
5-methyl-4-oxo-2-(pyrimidin-2-ylmethyl)-3,4-dihydrothieno-[2,3-d]pyrimidine-6-carboxylic acid,
2-(4-methoxybenzyl)-5-methyl-4-oxo-3,4-dihydrothieno[2,3-d]-pyrimidine-6-carboxylic acid,
2-(3,5-dichlorobenzyl)-5-methyl-4-oxo-3,4-dihydrothieno-[2,3-d]pyrimidine-6-carboxylic acid,
2-(4-chloro-3-fluorobenzyl)-5-methyl-4-oxo-3,4-dihydrothieno-[2,3-d]pyrimidine-6-carboxylic acid,
2-(4-chloro-3-methylbenzyl)-5-methyl-4-oxo-3,4-dihydrothieno[2,3-d]pyrimidine-6-carboxylic acid,
2-(4-chloro-3-trifluoromethylbenzyl)-5-methyl-4-oxo-3,4-dihydrothieno[2,3-d]pyrimidine-6-carboxylic acid,
2-(3-chloro-5-trifluoromethylbenzyl)-5-methyl-4-oxo-3,4-dihydrothieno[2,3-d]pyrimidine-6-carboxylic acid,
2-(3-carboxybenzyl)-5-methyl-4-oxo-3,4-dihydrothieno[2,3-d]-pyrimidine-6-carboxylic acid,
2-(3-ethoxycarbonylbenzyl)-5-methyl-4-oxo-3,4-dihydrothieno-[2,3-d]pyrimidine-6-carboxylic acid,
2-(3-aminobenzyl)-5-methyl-4-oxo-3,4-dihydrothieno[2,3-d]-pyrimidine-6-carboxylic acid,
5-methyl-2-(3-nitrobenzyl)-4-oxo-3,4-dihydrothieno[2,3-d]-pyrimidine-6-carboxylic acid,
3-amino-2-benzyl-5-methyl-4-oxo-3,4-dihydrothieno[2,3-d]-pyrimidine-6-carboxylic acid,
3-amino-5-methyl-4-oxo-2-(thiophen-2-ylmethyl)-3,4-dihydrothieno[2,3-d]pyrimidine-6-carboxylic acid,
3-amino-5-methyl-4-oxo-2-(thiophen-3-ylmethyl)-3,4-dihydrothieno[2,3-d]pyrimidine-6-carboxylic acid,
3-amino-2-(5-chlorothiophen-2-ylmethyl)-5-methyl-4-oxo-3, 4-dihydrothieno[2,3-d]pyrimidine-6-carboxylic acid,
3-amino-2-(2-fluorobenzyl)-5-methyl-4-oxo-3,4-dihydrothieno-[2,3-d]pyrimidine-6-carboxylic acid,
3-amino-2-(3-fluorobenzyl)-5-methyl-4-oxo-3,4-dihydrothieno-[2,3-d]pyrimidine-6-carboxylic acid,
3-amino-2-(4-fluorobenzyl)-5-methyl-4-oxo-3,4-dihydrothieno-[2,3-d]pyrimidine-6-carboxylic acid,
3-amino-2-(2-chlorobenzyl)-5-methyl-4-oxo-3,4-dihydrothieno-[2,3-d]pyrimidine-6-carboxylic acid,
3-amino-2-(3-chlorobenzyl)-5-methyl-4-oxo-3,4-dihydrothieno-[2,3-d]pyrimidine-6-carboxylic acid,
3-amino-2-(4-chlorobenzyl)-5-methyl-4-oxo-3,4-dihydrothieno-[2,3-d]pyrimidine-6-carboxylic acid,
3-amino-2-(3,4-dichlorobenzyl)-5-methyl-4-oxo-3,4-dihydrothieno[2,3-d]pyrimidine-6-carboxylic acid,
3-amino-2-(3-bromobenzyl)-5-methyl-4-oxo-3,4-dihydrothieno-[2,3-d]pyrimidine-6-carboxylic acid,
3-amino-5-methyl-2-(3-methylbenzyl)-4-oxo-3,4-dihydrothieno-[2,3-d]pyrimidine-6-carboxylic acid,
3-amino-5-methyl-4-oxo-2-(2-trifluoromethylbenzyl)-3,4-dihydrothieno[2,3-d]pyrimidine-6-carboxylic acid,
3-amino-5-methyl-4-oxo-2-(3-trifluoromethylbenzyl)-3,4-dlhydrothieno[2,3-d]pyrimidine-6-carboxylic acid,
3-amino-2-(cyclopenten-1-ylmethyl)-5-methyl-4-oxo-3,4-dihydrothieno[2,3-d]pyrimidine-6-carboxylic acid,
3-amino-2-(cyclohexen-1-ylmethyl)-5-methyl-4-oxo-3,4-dihydrothieno[2,3-d]pyrimidine-6-carboxylic acid,
2-(3-chlorobenzyl)-4-oxo-3,4-dihydroquinazoline-7-carboxylic acid,
2-(3,4-dichlorobenzyl)-4-oxo-3,4-dihydroquinazoline-7-carboxylic acid,
2-(3,4-dimethoxybenzyl)-4-oxo-3,4-dihydroquinazoline-7-carboxylic acid,
2-(2-methoxybenzyl)-4-oxo-3,4-dihydroquinazoline-7-carboxyic acid,
2-(3-methoxybenzyl)-4-oxo-3,4-dihydroquinazoline-7-carboxylic acid,
4-oxo-2-(2-pyridylmethyl)-3,4-dihydroquinazoline-7-carboxylic acid,
4-oxo-2-(3-pyridylmethyl)-3,4-dihydroquinazoline-7-carboxylic acid,
4-oxo-2-(2-thienylmethyl)-3,4-dihydroquinazoline-7-carboxylic acid,
2-benzyl-4-oxo-3,4-dihydroquinazoline-7-carboxylic acid,
2-(2-methylbenzyl)-4-oxo-3,4-dihydroquinazoline-7-carboxylic acid,
2-(3-methylbenzyl)-4-oxo-3,4-dihydroquinazoline-7-carboxylic acid,
2-(3-ethylbenzyl)-4-oxo-3,4-dihydroquinazoline-7-carboxylic acid,
2-(3-isopropylbenzyl)-4-oxo-3,4-dihydroquinazoline-7-carboxylic acid,
2-(4-methylbenzyl)-4-oxo-3,4-dihydroquinazoline-7-carboxylic acid,
2-(4-methoxybenzyl)-4-oxo-3,4-dihydroquinazoline-7-carboxylic acid,

2-(2-ethoxybenzyl)-4-oxo-3,4-dihydroquinazoline-7-carboxylic acid,
2-(3-ethoxybenzyl)-4-oxo-3,4-dihydroquinazoline-7-carboxylic acid,
2-(4-ethoxybenzyl)-4-oxo-3,4-dihydroquinazoline-7-carboxylic acid,
2-(3-tert-butoxybenzyl)-4-oxo-3,4-dihydroquinazoline-7-carboxylic acid,
4-oxo-2-(2-trifluoromethylbenzyl)-3,4-dihydroquinazoline-7-carboxylic acid,
4-oxo-2-(3-trifluoromethylbenzyl)-3,4-dihydroquinazoline-7-carboxylic acid,
4-oxo-2-(4-trifluoromethylbenzyl)-3,4-dihydroquinazoline-7-carboxylic acid,
2-(2-fluorobenzyl)-4-oxo-3,4-dihydroquinazoline-7-carboxylic acid,
2-(3-fluorobenzyl)-4-oxo-3,4-dihydroquinazoline-7-carboxylic acid,
2-(4-fluorobenzyl)-4-oxo-3,4-dihydroquinazoline-7-carboxylic acid,
2-(2-bromobenzyl)-4-oxo-3,4-dihydroquinazoline-7-carboxylic acid,
2-(3-bromobenzyl)-4-oxo-3,4-dihydroquinazoline-7-carboxylic acid,
2-(4-bromobenzyl)-4-oxo-3,4-dlhydroquinazoline-7-carboxylic acid,
2-(2-chlorobenzyl)-4-oxo-3,4-dihydroquinazoline-7-carboxylic acid,
2-(3-chlorobenzyl)-4-oxo-3,4-dihydroquinazoline-7-carboxylic acid,
2-(4-chlorobenzyl)-4-oxo-3,4-dihydroquinazoline-7-carboxylic acid,
2-(4-fluoro-3-trifluoromethylbenzyl)-4-oxo-3,4-dihydroquinazoline-7-carboxylic acid,
2-(2,3-difluorobenzyl)-4-oxo-3,4-dihydroquinazoline-7-carboxylic acid,
2-(2,4-difluorobenzyl)-4-oxo-3,4-dihydroquinazoline-7-carboxylic acid,
2-(2,5-difluorobenzyl)-4-oxo-3,4-dihydroquinazoline-7-carboxylic acid,
2-(2,6-difluorobenzyl)-4-oxo-3,4-dihydroquinazoline-7-carboxylic acid,
2-(3,4-difluorobenzyl)-4-oxo-3,4-dihydroquinazoline-7-carboxylic acid,
2-(3-chloro-2-fluorobenzyl)-4-oxo-3,4-dihydroquinazoline-7-carboxylic acid,
2-(4-chloro-2-fluorobenzyl)-4-oxo-3,4-dihydroquinazoline-7-carboxylic acid,
2-(5-chloro-2-fluorobenzyl)-4-oxo-3,4-dihydroquinazoline-7-carboxylic acid,
2-(4-chloro-3-fluorobenzyl)-4-oxo-3,4-dihydroquinazoline-7-carboxylic acid,
2-(2-chloro-4-fluorobenzyl)-4-oxo-3,4-dihydroquinazoline-7-carboxylic acid,
2-(3-chloro-4-fluorobenzyl)-4-oxo-3,4-dihydroquinazoline-7-carboxylic acid,
2-(3-bromo-4-fluorobenzyl)-4-oxo-3,4-dihydroquinazoline-7-carboxylic acid,
2-(2,6-dichlorobenzyl)-4-oxo-3,4-dihydroquinazoline-7-carboxylic acid,
2-(2,5-dichlorobenzyl)-4-oxo-3,4-dihydroquinazoline-7-carboxylic acid,
2-(3,5-dichlorobenzyl)-4-oxo-3,4-dihydroquinazoline-7-carboxylic acid,
4-oxo-2-(2,3,4-trifluorobenzyl)-3,4-dihydroquinazoline-7-carboxylic acid,
4-oxo-2-(2,4,5-trifluorobenzyl)-3,4-dihydroquinazoline-7-carboxylic acid,
4-oxo-2-(2,4,6-trifluorobenzyl)-3,4-dihydroquinazoline-7-carboxylic acid,
4-oxo-2-(3,4,5-trifluorobenzyl)-3,4-dihydroquinazoline-7-carboxylic acid,
2-(2-fluoro-4-methoxybenzyl)-4-oxo-3,4-dihydroquinazoline-7-carboxylic acid,
2-(4-fluoro-3-methoxybenzyl)-4-oxo-3,4-dihydroquinazoline-7-carboxylic acid,
2-(2,3-dimethoxybenzyl)-4-oxo-3,4-dihydroquinazoline-7-carboxylic acid,
2-(2,6-dimethoxybenzyl)-4-oxo-3,4-dihydroquinazoline-7-carboxylic acid,
2-(2-fluoro-5-methylbenzyl)-4-oxo-3,4-dihydroquinazoline-7-carboxylic acid,
2-(3-fluoro-4-methylbenzyl)-4-oxo-3,4-dihydroquinazoline-7-carboxylic acid,
2-(4-fluoro-3-methylbenzyl)-4-oxo-3,4-dihydroquinazoline-7-carboxylic acid,
2-(4-chloro-3-methylbenzyl)-4-oxo-3,4-dihydroquinazoline-7-carboxylic acid,
2-(4-methoxy-3-methylbenzyl)-4-oxo-3,4-dihydroquinazoline-7-carboxylic acid,
2-(2,3-dimethylbenzyl)-4-oxo-3,4-dihydroquinazoline-7-carboxylic acid,
2-(2,5-dimethylbenzyl)-4-oxo-3,4-dihydroquinazoline-7-carboxylic acid,
2-(3,4-dimethylbenzyl)-4-oxo-3,4-dihydroquinazoline-7-carboxylic acid,
2-(3,5-dimethylbenzyl)-4-oxo-3,4-dihydroquinazoline-7-carboxylic acid,
2-(2,6-dimethylbenzyl)-4-oxo-3,4-dihydroquinazoline-7-carboxylic acid,
2-[2-(6-chloropyridylmethyl)]-4-oxo-3,4-dihydroquinazoline-7-carboxylic acid,
2- [2-(5, 6-dichloropyridylmethyl)] -4-oxo-3,4-dihydroquinazoline-7-carboxylic acid,
2-[3-(6-chloropyridylmethyl)]-4-oxo-3,4-dihydroquinazoline-7-carboxylic acid,
2-[3-(5,6-dichloropyridylmethyl)]-4-oxo-3,4-dihydroquinazoline-7-carboxylic acid,
2-[2-(6-methoxypyridylmethyl)]-4-oxo-3,4-dihydroquinazoline-7-carboxylic acid,
2-[2-(5,6-dimethoxypyridylmethyl)]-4-oxo-3,4-dihydroquinazoline-7-carboxylic acid,
2-[3-(6-methoxypyridylmethyl)]-4-oxo-3,4-dihydroquinazoline-7-carboxylic acid,
2-[3-(5,6-dimethoxypyridylmethyl)]-4-oxo-3,4-dihydroquinazoline-7-carboxylic acid,

4-oxo-2-(3-thienylmethyl)-3,4-dihydroquinazoline-7-carboxylic acid,
2-[2-(5-chlorothienylmethyl)]-4-oxo-3,4-dihydroquinazoline-7-carboxylic acid,
2-[2-(5-methoxythienylmethyl)]-4-oxo-3,4-dihydroquinazoline-7-carboxylic acid,
2-benzyl-4-oxo-3,4-dihydroquinazoline-6-carboxylic acid,
2-(3,4-dichlorobenzyl)-4-oxo-3,4-dihydroquinazoline-6-carboxylic acid,
2-(3,4-dimethoxybenzyl)-4-oxo-3,4-dihydroquinazoline-6-carboxylic acid,
2-(2-methoxybenzyl)-4-oxo-3,4-dihydroquinazoline-6-carboxylic acid,
2-(3-methoxybenzyl)-4-oxo-3,4-dihydroquinazoline-6-carboxylic acid,
2-(3-chlorobenzyl)-4-oxo-3,4-dihydroquinazoline-6-carboxylic acid,
4-oxo-2-(2-pyridylmethyl)-3,4-dihydroquinazoline-6-carboxylic acid,
4-oxo-2-(3-pyridylmethyl)-3,4-dihydroquinazoline-6-carboxylic acid,
4-oxo-2-(2-thienylmethyl)-3,4-dihydroquinazoline-6-carboxylic acid,
2-benzyl-4-oxo-3,4-dihydroquinazoline-5-carboxylic acid,
2-(3,4-dichlorobenzyl)-4-oxo-3,4-dihydroquinazoline-5-carboxylic acid,
2-(3,4-dimethoxybenzyl)-4-oxo-3,4-dihydroquinazoline-5-carboxylic acid,
2-(2-methoxybenzyl)-4-oxo-3,4-dihydroquinazoline-5-carboxylic acid,
2-(3-methoxybenzyl)-4-oxo-3,4-dihydroquinazoline-5-carboxylic acid,
2-(3-chlorobenzyl)-4-oxo-3,4-dihydroquinazoline-5-carboxylic acid,
4-oxo-2-(2-pyridylmethyl)-3,4-dihydroquinazoline-5-carboxylic acid,
4-oxo-2-(3-pyridylmethyl)-3,4-dihydroquinazoline-5-carboxylic acid,
4-oxo-2-(2-thienylmethyl)-3,4-dihydroquinazoline-5-carboxylic acid,
2-benzyl-4-oxo-3,4-dihydroquinazoline-8-carboxylic acid,
2-(3,4-dichlorobenzyl)-4-oxo-3,4-dihydroquinazoline-8-carboxylic acid,
2-(3,4-dimethoxybenzyl)-4-oxo-3,4-dihydroquinazoline-8-carboxylic acid,
2-(2-methoxybenzyl)-4-oxo-3,4-dihydroquinazoline-8-carboxylic acid,
2-(3-methoxybenzyl)-4-oxo-3, 4-dihydroquinazoline-8-carboxylic acid,
2-(3-chlorobenzyl)-4-oxo-3,4-dihydroquinazoline-8-carboxylic acid,
4-oxo-2-(2-pyridylmethyl)-3,4-dihydroquinazoline-8-carboxylic acid,
4-oxo-2-(3-pyridylmethyl)-3,4-dihydroquinazoline-8-carboxylic acid,
4-oxo-2-(2-thienylmethyl)-3,4-dihydroquinazoline-8-carboxylic acid,
4-oxo-2-phenethyl-3,4-dihydroquinazoline-7-carboxylic acid,
2-(3,4-dichlorophenethyl)-4-oxo-3,4-dihydroquinazoline-7-carboxylic acid,
2-(3,4-dimethoxyphenethyl)-4-oxo-3,4-dihydroquinazoline-7-carboxylic acid,
2-(2-methoxyphenethyl)-4-oxo-3,4-dihydroquinazoline-7-carboxylic acid,
2-(3-methoxyphenethyl)-4-oxo-3,4-dihydroquinazoline-7-carboxylic acid,
2-(3-chlorophenethyl)-4-oxo-3,4-dihydroquinazoline-7-carboxylic acid,
4-oxo-2-[2-(2-pyridyl)ethyl]-3,4-dihydroquinazoline-7-carboxylic acid,
4-oxo-2-[2-(3-pyridyl)ethyl]-3,4-dihydroquinazoline-7-carboxylic acid,
4-oxo-2-[2-(2-thienyl)ethyl]-3,4-dihydroquinazoline-7-carboxylic acid,
4-oxo-2-(3-phenylpropyl)-3,4-dihydroquinazoline-7-carboxylic acid,
2-[3-(3,4-dimethoxyphenyl)propyl]-4-oxo-3,4-dihydroquinazoline-7-carboxylic acid,
2-[3-(2-methoxyphenyl)propyl]-4-oxo-3,4-dihydroquinazoline-7-carboxylic acid,
2-[3-(3-methoxyphenyl)propyl]-4-oxo-3,4-dihydroquinazoline-7-carboxylic acid,
2-[3-(3-chlorophenyl)propyl]-4-oxo-3,4-dihydroquinazoline-7-carboxylic acid,
4-oxo-2-[3-(2-pyridyl)propyl)-3,4-dihydroquinazoline-7-carboxylic acid,
4-oxo-2-[3-(3-pyridyl)propyl]-3,4-dihydroquinazoline-7-carboxylic acid,
4-oxo-2-[3-(2-thienyl)propyl]-3,4-dihydroquinazoline-7-carboxylic acid,
7-chloro-1-isopropylimidazo[1,5-a]quinoxalin-4(5H)-one,
1-isopropyl-N,N-dimethyl-4-oxo-4,5-dihydroimidazo[1,5-a]-quinoxaline-7-carboxamide,
1-cyclohexyl-8-methoxy-N,N-dimethyl-4-oxo-4,5-dihydroimidazo[1,5-a]quinoxaline-7-carboxamide,
1-cyclohexyl-8-ethoxy-N,N-dimethyl-4-oxo-4,5-dihydroimidazo-[1,5-a]quinoxaline-7-carboxamide,
8-chloro-1-cyclohexyl-N,N-dimethyl-4-oxo-4,5-dihydroimidazo-[1,5-a]quinoxaline-7-carboxamide,
1-[(8-chloro-1-cyclohexyl-4-oxo-4,5-dihydroimidazo[1,5-a]-quinoxalin-7-yl)carbonyl]-4-(2-hydroxyethyl)piperazine,
3-(7-ethyl-4-oxo-4,5-dihydroimidazo[1,5-a]quinoxalin-1-yl)-propionic acid,
3-(4-oxo-7-trifluoromethyl-4,5-dihydroimidazo[1,5-a]-quinoxalin-1-yl)propionic acid,
3-(7-bromo-4-oxo-4,5-dihydroimidazo[1,5-a]quinoxalin-1-yl)-propionic acid,
(E)-3-(4-oxo-7-trifluoromethyl-4,5-dihydropyrrolo[1,2-a]-quinoxalin-1-yl)acrylic acid,
N,N-dimethyl-4-oxo-1-(tetrahydropyran-4-yl)-4,5-dihydroimidazo[1,5-a]quinoxaline-8-carboxamide,

N,N-3-trimethyl-4-oxo-1-(tetrahydropyran-4-yl)-4,5-dihydroimidazo[1,5-a]quinoxaline-8-carboxamide,
N,N-dimethyl-4-oxo-1-(tetrahydrothiopyran-4-yl)-4,5-dihydroimidazo[1,5-a]quinoxaline-8-carboxamide,
N,N-dimethyl-1-[4-(1-methylpiperidyl)-4-oxo-4,5-dihydroimidazo[1,5-a]quinoxaline-8-carboxamide,
1-cyclohexyl-N-methyl-4-oxo-N-trifluoromethyl-4,5-dihydroimidazo[1,5-a]quinoxaline-8-carboxamide,
1-cyclohexyl-4-oxo-N,N-bis(trifluoromethyl)-4,5-dihydroimidazo[1,5-a]quinoxaline-8-carboxamide,
1-cyclohexyl-N-(2-ethoxyethyl)-N-methyl-4-oxo-4,5-dihydroimidazo[1,5-a]quinoxaline-8-carboxamide,
1-cyclohexyl-N-methyl-4-oxo-N-(2-propoxyethyl)-4,5-dihydroimidazo[1,5-a]quinoxaline-8-carboxamide,
1-cyclohexyl-N-(2-isopropoxyethyl)-N-methyl-4-oxo-4,5-dihydroimidazo[1,5-a]quinoxaline-8-carboxamide,
1-cyclohexyl-N-(3-methoxypropyl)-N-methyl-4-oxo-4,5-dihydroimidazo[1,5-a]quinoxaline-8-carboxamide,
1-cyclohexyl-N-(2-methoxyethyl)-4-oxo-N-trifluoromethyl-4,5-dihydroimidazo[1,5-a]quinoxaline-8-carboxamide,
1-cyclohexyl-N-(2-methoxyethyl)-3-methyl-4-oxo-N-trifluoromethyl-4,5-dihydroimidazo[1,5-a]quinoxaline-8-carboxamide,
1-cyclohexyl-N-methyl-4-oxo-N-(2-trifluoromethoxyethyl)-4,5-dihydroimidazo[1,5-a]quinoxaline-8-carboxamide,
1-cyclohexyl-N,3-dimethyl-4-oxo-N-(2-trifluoromethoxyethyl)-4,5-dihydroimidazo[1,5-a]quinoxaline-8-carboxamide,
1-cyclohexyl-N-methyl-N-[4-(1-methylpiperidyl)]-4-oxo-4,5-dihydroimidazo[1,5-a]quinoxaline-8-carboxamide,
1-cyclohexyl-N-methyl-4-oxo-N-(2-pyridyl)-4,5-dihydroimidazo[1,5-a]quinoxaline-8-carboxamide,
1-cyclohexyl-N-methyl-4-oxo-N-(3-pyridyl)-4,5-dihydroimidazo[1,5-a]quinoxaline-8-carboxamide,
1-cyclohexyl-N-methyl-4-oxo-N-(4-pyridyl)-4,5-dihydroimidazo[1,5-a]quinoxaline-8-carboxamide,
1-cyclohexyl-N-methyl-4-oxo-N-(2-pyridylmethyl)-4,5-dihydroimidazo[1,5-a]quinoxaline-8-carboxamide,
1-cyclohexyl-N-methyl-4-oxo-N-(3-pyridylmethyl)-4,5-dihydroimidazo[1,5-a]quinoxaline-8-carboxamide,
1-cyclohexyl-N-methyl-4-oxo-N-(4-pyridylmethyl)-4,5-dihydroimidazo[1,5-a]quinoxaline-8-carboxamide,
1-cyclohexyl-N,N-dimethyl-4-oxo-8-trifluoromethoxy-4,5-dihydroimidazo[1,5-a]quinoxaline-7-carboxamide,
5-(3-chlorobenzyl)-3-isopropyl-1,6-dihydropyrazolo[4,3-d]-pyrimidin-7-one,
3-pyridin-3-yl-5-(2,6-dichlorobenzyl)-1,6-dihydropyrazolo-[4,3-d]pyrimidin-7-one,
3-butyl-5-(2-methoxybenzyl)-1,6-dihydropyrazolo[4,3-d]-pyrimidin-7-one,
3-tert-butyl-5-(3-chlorobenzyl)-1,6-dihydropyrazolo[4,3-d]-pyrimidin-7-one,
3-isopropyl-5-(2-phenoxybenzyl)-1,6-dihydropyrazolo[4,3-d]-pyrimidin-7-one,
3-pyridin- 3-yl-5-(2-benzyloxybenzy1)-1,6-dihydropyrazolo-[4,3-d]pyrimidin-7-one,
3-isopropyl-5-(2-trifluoromethoxybenzyl)-1,6-dihydropyrazolo-[4,3-d]pyrimidin-7-one,
3-cyclopentyl-5-(2-benzyloxybenzyl)-1,6-dihydropyrazolo-[4,3-d]pyrimidin-7-one,
3-pyridin-3-yl-5-(2-trifluoromethylbenzyl)-1,6-dihydropyrazolo[4,3-d]pyrimidin-7-one,
3-cyclopentyl-5-(2-trifluoromethoxybenzyl)-1,6-dihydropyrazolo[4,3-d]pyrimidin-7-one,
3-pyridin-3-yl-5-(2-trifluoromethoxybenzyl)-1,6-dihydropyrazolo[4,3-d]pyrimidin-7-one,
3-cyclopentyl-5-(2,4,6-trifluorobenzyl)-1,6-dihydropyrazolo-[4,3-d]pyrimidin-7-one,
5-cyclopentylmethyl-3-isobutyl-1,6-dihydropyrazolo[4,3-d]-pyrimidin-7-one,
6-(3-chlorobenzyl)-1-cyclopentyl-1,5-dihydropyrazolo[3,4-d]-pyrimidin-4-one,
6-(2-fluorobenzyl)-1-cyclopentyl-1,5-dihydropyrazolo[3,4-d]-pyrimidin-4-one,
6-(3,4-dichlorobenzyl)-1-cyclopentyl-1,5-dihydropyrazolo-[3,4-d]pyrimidin-4-one,
6-(3-methylbenzyl)-1-(1-ethylpropyl)-1,5-dihydropyrazolo-[3,4-d]pyrimidin-4-one,
6-(3-chlorobenzyl)-1-cyclopentyl-1,5-dihydropyrazolo[3,4-d]-pyrimidine-4-thione, and the like.

Those compounds which are used as the active ingredients in the treating agent of the present invention may be present in the form of their salts. As the salts, for example, alkali metal salts such as sodium salt, potassium salt, lithium salt and the like; alkaline earth metal salts such as calcium salt, magnesium salt and the like; salts with organic bases such as triethylamine, dicyclohexylamine, pyrrolidine, morpholine, pyridine and the like; ammonium salts and the like. Furthermore, depending on the kind(s) of the substituent(s), they may form salts with inorganic acids such as hydrochloric acid, hydrobromic acid, sulfuric acid, nitric acid, phosphoric acid and the like; and salts with organic acids such as acetic acid, oxalic acid, citric acid, lactic acid, tartaric acid, p-toluenesulfonic acid and the like. Of these salts, pharmaceutically acceptable salts are particularly preferred.

Those compounds of the formulae (I), (II) and (III) can be easily prepared by the production methods as given in the later-appearing Production Examples, or by the methods known per se, for example, those described in SYNTHESIS, 1980, 150-151, Bioorg. Med. Chem. Lett., 2002 (12), 1275-1278, and so on. Also the compounds of the formula (IV) and formula (V) are described, as aforesaid, in PCT International Publications WO 2004/018474 Pamphlet and WO 03/037899 Pamphlet, respectively.

Those treating agents of the present invention possess excellent PDE9-inhibiting activity and are useful for therapy or treatment of diseases associated with degradation of cGMP by PDE9, for example, overactive bladder syndrome, pollakiuria, urinary incontinence, dysuria in benign prostatic hyperplasia, neurogenic bladder, interstitial cystitis, urolithiasis, benign prostatic hyperplasia, erectile dysfunction, cognitive impairment, neuropathy, Alzheimer's disease, pulmonary hypertension, chronic obstructive pulmonary disease, ischemic heart disease, hypertension, angina, myocardial infarction, arteriosclerosis, thrombosis, embolism, type I diabetes and type II diabetes. The treating agents of the present invention are particularly useful for therapy or treatment of, among those diseases, overactive bladder syndrome, pollakiuria, urinary incontinence, dysuria in benign prostatic hyperplasia and urolithiasis, inter alia, exhibit excellent efficacy in the therapy or treatment of overactive bladder syndrome, pollakiuria, urinary incontinence and dysuria in benign prostatic hyperplasia.

Some of the compounds or salts thereof which are used in the treating agents of the present invention exhibit slight PDE5-inhibiting activity in addition to the PDE9-inhibiting activity. The treating agents containing such compounds or salts are expected to achieve also the functional effects based on the PDE5-inhibiting activity.

PDE9-inhibiting activity and PDE5-inhibiting activity possessed by the compounds or their salts used in the treating agents of the present invention, and their efficacy for dysuria pathological model are demonstrated by the following experiments.

(1) Measurement of PDE9-inhibiting activity:

1) Preparation of human recombinant PDE9 protein

Based on the base sequence of hsPDE9A1 registered with GenBank database (accession No.: AF048837), hsPDE9A1 fragment was amplified by polymerase chain reaction under the following conditions, using the following sequence (Amasham Pharmacia Biotech) as the primer and Human Prostate MATCHMAKER cDNA library (CLONTECH) as the template DNA, with Pfu Turbo DNA polymerase (STRATAGENE):

hPDE9-5A primer: CCTAGCTAGCCACCATGGGATCCGGCTCCTCC
hPDE9-3A primer: TTTTCCTTTTGCGGCCGCTTATTAGGCACAGTCTCCTTCACTG
PCR condition: [95°C, 5 min] ×1 cycle, [(95°C, 1 min), (58°C, 2 min), (72°C, 3 min)] × 25 cycles, [72°C, 10 min] × 1 cycle

Thus obtained hsPDE9A1 fragment was given a restricted enzymatic treatment with NheI and NotI, and thereafter inserted into pcDNA 3.1(+) expression vector (Invitrogen) to let it serve as a human PDE9 expression vector.

Human PDE9 expression vector-transformed Escherichia coli was mass incubated to produce a large amount of human PDE9 expression vector, which was transiently transfected into COS-1 cells, with LIPOFECTAMINE 2000 Reagent (GIBCO). The cells were homogenized in ice-cooled buffer A (40 mmol/L Tris-HCl, pH7.5, 15 mmol/L benzamidine, 15 mmol/L 2-mercaptoethanol, 1 μg/mL Pepstatin A, 1 μg/mL Leupeptin, 5 mmol/L EDTA) and centrifuged at 4°C, 14,000 x g for 10 minutes. The supernatant was isolated to provide human recombinant PDE9 protein solution.

2) Measurement of PDE9-inhibiting activity

To 150 μL of buffer B (70 mmol/L Tris-HCl, pH7.5, 16.7 mmol/L $MgCl_2$, 33.3 nmol/L [$^3$H]-cGMP) solution containing [$^3$H]-cGMP (specific activity = 244.2 GBq/mmol) at a concentration of 33.3 nmol/L, 50 μL of a solution of the compound to be evaluated (formed by dissolving the compound in DMSO and diluting it with distilled water to DMSO concentration of 5%) and 50 μL of the PDE9 protein solution as prepared in the above, as diluted with buffer C (40 mmol/L Tris-HCl, pH7.5, 15 mmol/L benzamidine, 15 mmol/L 2-mercaptoethanol, 1 μg/mL Pepstatin A, 1 μg/mL Leupeptin) by 1,500X, were added under cooling with ice. This mixed solution was incubated at 30°C for 30 minutes and the enzymatic reaction of PDE9 was terminated by heating the system in boiling water for 90 seconds. Returning the system to room temperature, 50 μL of Snake venom (SIGMA: 1 mg/mL) was added, followed by 10 minutes' incubation at 30°C, to convert the [$^3$H]-5'-GMP produced in the previous reaction to [$^3$H]-guanosine. This reaction solution was passed through a column filled with 1 mL of 0.5 mol/L hydrochloric acid-activated cation-exchange resin (Bio-Rad AG50W - X4 resin, mesh size 200 - 400) and removed of the unreacted substrate ([$^3$H]-cGMP) by elution with 12 mL of distilled water. Thereafter [$^3$H]-guanosine was eluted with 3 mL of 3 mol/L aqueous ammonia and its radiation activity was measured with liquid scintillation counter.

PDE9 inhibition of the tested compound can be calculated by the following formula:

$$\left[\left(1- \frac{\text{radiation activity where a test compound is used}}{\text{radiation activity in control test}}\right) \times 100\right].$$

From the percent inhibition at various concentration levels of each tested compound, its $IC_{50}$ value against PDE9 can be determined. The results are shown in Tables A-C given later.

(2) Measurement of PDE5-inhibiting activity:

1) Preparation of human recombinant PDE5 protein

Based on the base sequence of hsPDE5A1 registered with GenBank database (accession No.: NM-001083), hsPDE5A1 fragment was amplified by polymerase chain reaction (PCR) under the following conditions, using the following sequence (SIGMA GENOSYS) as the primer and Human Prostate MATCHMAKER cDNA library (CLONTECH) as the template DNA, with KDD plus DNA polymerase (TOYOBO):

hPDE5-5' E primer: CGGAATTCCAACCATGGAGCGGGC
hPDE5-3' primer: GCTCTAGATCAGTTCCGCTTGGCCTGG
PCR condition: [94˚C, 2 min] × 1 cycle, [(94˚C, 30 sec), (65˚C,30 sec), (68˚C, 3 min)] × 25 cycles, [68˚C,6 min] × 1 cycle

Thus obtained hsPDE5A1 fragment was given a restricted enzymatic treatment with XBaI and EcoRI, and thereafter inserted into pcDNA 3.1(+) expression vector (Invitrogen) to let it serve as a human PDE5 expression vector.
Human PDE5 expression vector-transformed Escherichia coli was mass incubated to produce a large amount of human PDE5 expression vector, which was transiently transfected into COS-1 cells, with LIPOFECTAMINE 2000 Reagent (GIBCO). The cells were homogenized in ice-cooled buffer A and centrifuged at 4˚C, 14,000 x g for 10 minutes. The supernatant was isolated to provide human recombinant PDE5 protein solution.

2) Measurement of PDE5-inhibiting activity

By a method similar to the measurement of PDE9-inhibiting activity, PDE5-inhibiting activity of each of the test compounds was measured, percent inhibition was calculated and $IC_{50}$ value against PDE5 was determined. The results are shown in the following Tables A-C, concurrently with the compounds' PDE9-inhibiting activity. The compound of Compound No. C-11 in the later-appearing Tables C and D is posted on PCT International Publication WO 03/037899 Pamphlet which discloses compounds of the formula (V).

TABLE A

| Compound No. | Structural Formula | Inhibiting Activity ($IC_{50}$ value: nmol/L) | |
|---|---|---|---|
| | | PDE9 | PDE5 |
| A-1 | 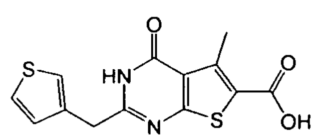 | 22 | 17,784 |
| A-2 | 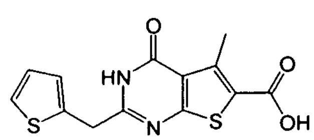 | 40 | 21,116 |
| A-3 | 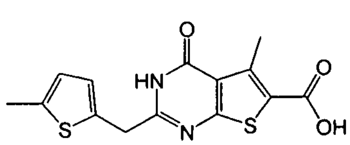 | 34 | 6,897 |
| A-4 | 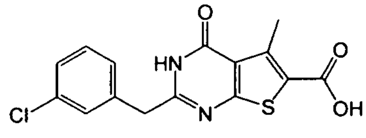 | 30 | 6,767 |

(continued)

| Compound No. | Structural Formula | Inhibiting Activity ($IC_{50}$ value: nmol/L) | |
|---|---|---|---|
| | | PDE9 | PDE5 |
| A-5 | 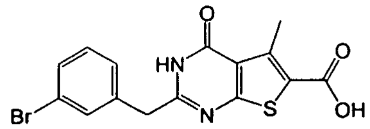 | 24 | 4,430 |
| A-6 | 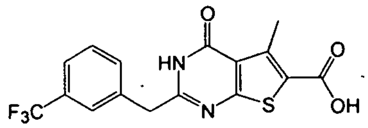 | 34 | 22,159 |
| A-7 | 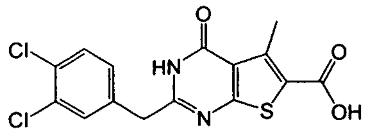 | 38 | 915 |
| A-8 | 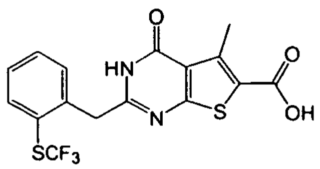 | 46 | 20,008 |
| A-9 | 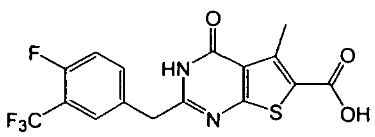 | 44 | 18,903 |
| A-10 | 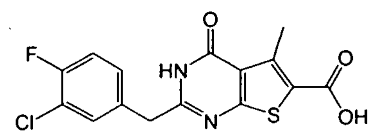 | 38 | 3,417 |
| A-11 | 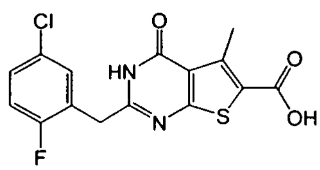 | 22 | 5,712 |
| A-12 | 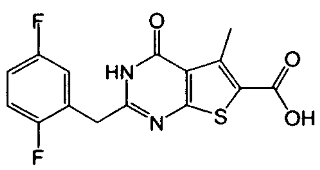 | 42 | 19,834 |
| A-13 | 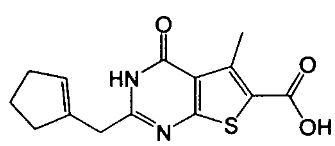 | 28 | 8,591 |

(continued)

| Compound No. | Structural Formula | Inhibiting Activity ($IC_{50}$ value: nmol/L) | |
|---|---|---|---|
| | | PDE9 | PDE5 |
| A-14 | 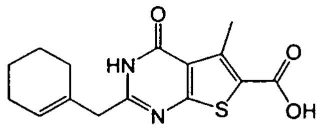 | 54 | 1,638 |
| A-15 | 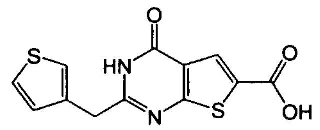 | 14 | 34,879 |
| A-16 | 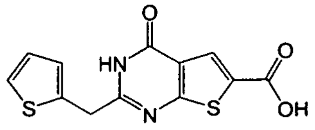 | 15 | 41,232 |
| A-17 | 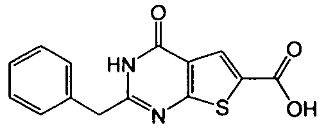 | 19 | 34,389 |
| A-18 | 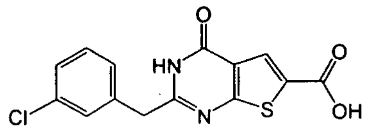 | 10 | 15,819 |
| A-19 | 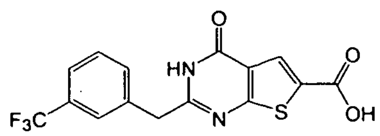 | 15 | 30,222 |
| A-20 | 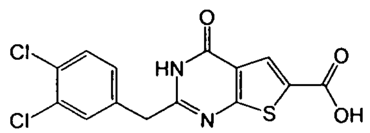 | 9 | 2,282 |
| A-21 | 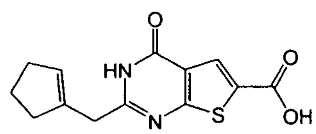 | 22 | 12,065 |
| A-22 | 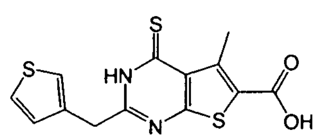 | 9 | 1,636 |
| A-23 | 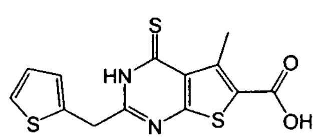 | 31 | 1,541 |

(continued)

| Compound No. | Structural Formula | Inhibiting Activity ($IC_{50}$ value: nmol/L) | |
|---|---|---|---|
| | | PDE9 | PDE5 |
| A-24 | 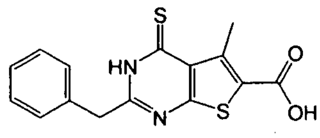 | 13 | 642 |
| A-25 | 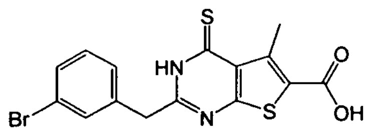 | 11 | 712 |
| A-26 | 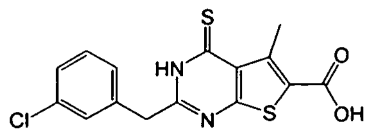 | 5 | 1,112 |
| A-27 | 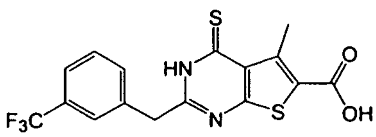 | 6 | 3,507 |
| A-28 | 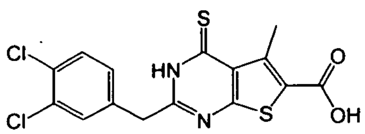 | 4 | 73 |
| A-29 | 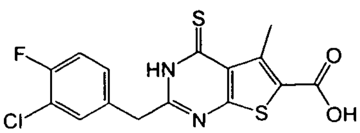 | 7 | 495 |
| A-30 | 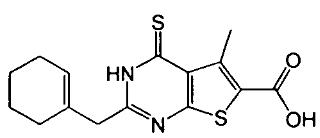 | 48 | 70 |
| A-31 | 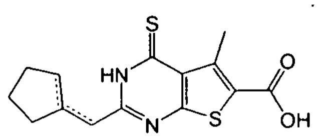 | 24 | 843 |
| A-32 | 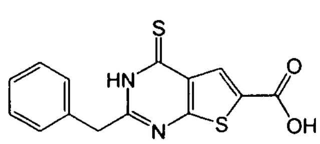 | 11 | 8,874 |

(continued)

| Compound No. | Structural Formula | Inhibiting Activity ($IC_{50}$ value: nmol/L) | |
|---|---|---|---|
| | | PDE9 | PDE5 |
| A-33 | 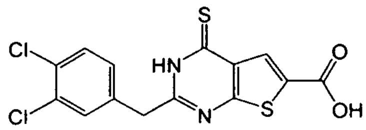 | 5 | 721 |
| A-34 | 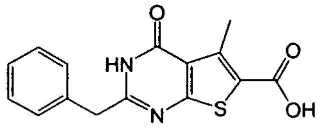 | 35 | 10,045 |

TABLE B

| Compound No. | Structural Formula | Inhibiting Activity ($IC_{50}$ value or inhibition ratio at 1$\mu$M) | |
|---|---|---|---|
| | | PDE9 | PDE5 |
| B-1 | 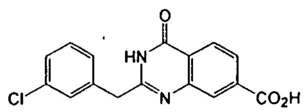 | $IC_{50}$=18nM | $IC_{50}$=6,210nM |
| B-2 | 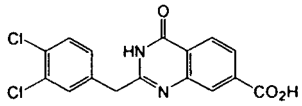 | $IC_{50}$=35nM | $IC_{50}$=1,435nM |
| B-3 | 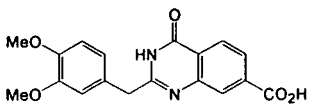 | inhibition ratio=11% | - |
| B-4 | 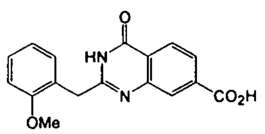 | $IC_{50}$=1,290nM | - |
| B-5 | 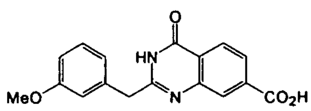 | inhibition ratio=41% | - |
| B-6 | 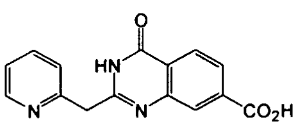 | $IC_{50}$=1,360nM | - |
| B-7 | 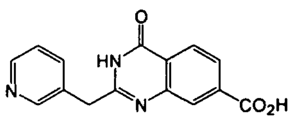 | inhibition ratio=30% | - |

(continued)

| Compound No. | Structural Formula | Inhibiting Activity ($IC_{50}$ value or inhibition ratio at 1μM) | |
|---|---|---|---|
| | | PDE9 | PDE5 |
| B-8 | 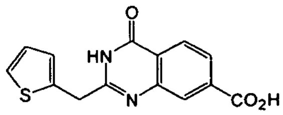 | $IC_{50}$=93nM | - |
| B-9 | 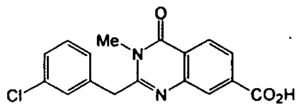 | $IC_{50}$=4,659nM | - |

TABLE C

| Compound No. | Structural Formula | Inhibiting Activity ($IC_{50}$ value: nmol/L) | |
|---|---|---|---|
| | | PDE9 | PDE5 |
| C-1 | 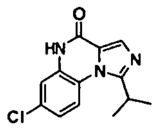 | 10 | 1,259 |
| C-2 | 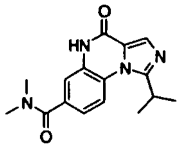 | 42 | > 10,000 |
| C-3 | 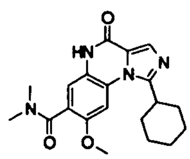 | 1.2 | > 10,000 |
| C-4 | 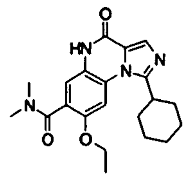 | 3.4 | > 10,000 |
| C-5 | 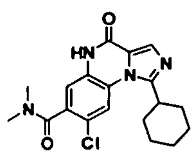 | 3.0 | > 10,000 |
| C-6 | 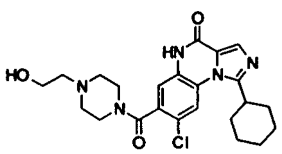 | 8.0 | 7,544 |

(continued)

| Compound No. | Structural Formula | Inhibiting Activity ($IC_{50}$ value: nmol/L) | |
|---|---|---|---|
| | | PDE9 | PDE5 |
| C-7 | 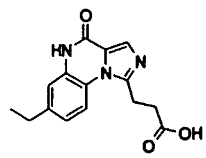 | 2.0 | > 10,000 |
| C-8 | 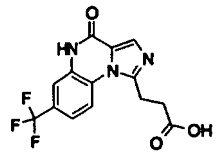 | 2.0 | 6,371 |
| C-9 | 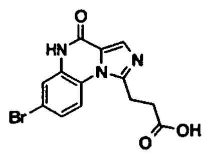 | 3.0 | 2,814 |
| C-10 | 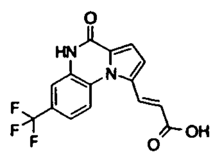 | 8.0 | 4,561 |
| C-11 | 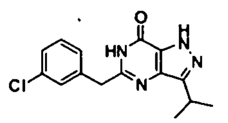 | 8.0 | 1,337 |

(3) Investigation of PDE9-inhibiting activity on dysuria pathological model

Three-four weeks old Hartley female guinea pigs (Japan SLC, Inc.) were given celiotomy under anesthesia with pentobarbital (30 mg/kg i.p.) and in their urethra to the peripheral side by 1-2 mm from the bladder neck, each a polyethylene tube of 1.4 mm in width and 2.0 mm in inner diameter was placed. After closing the wound, the guinea pigs were bred for at least 3 weeks to produce partial urethra obstruction model in guinea pigs in which occurrence of intravesical pressure rise not accompanied by micturition (uninhibited contraction) and residual urine were observed.

The model was catheterized under anesthesia with urethane (1 g/kg i.p.) at the apex of urinary bladder and right jugular vein for cystometrography and intravenous administration, respectively. The other end of the bladder catheter was connected to a pressure transducer and infusion pump via a three way stop-cock, and physiological saline was continuously infused into the bladder through the infusion pump at a rate of 0.4 mL/min to induce micturition reflex. Immediately after the micturition reflex was induced, infusion of physiological saline into the bladder was stopped. The intravesical pressure at the time the micturition reflex was induced was measured with the pressure transducer, and the obtained cystometrogram was recorded with pen recorder. The urine voided was collected with disposable type weighing dish and its weight was measured. Further the physiological saline remained in the bladder was sucked with syringe via the bladder catheter and the residual urine volume was measured. The operations of suspending the infusion of physiological saline upon induction of micturition reflex, and about 1 minute thereafter resuming the infusion of physiological saline to induce micturition reflex were repeated plural times (generally 4 times) to stabilize the micturition reflex.

After stabilizing the micturition reflex, a compound solution (prepared by dissolving the compound or methanesulfonic acid salt thereof in DMSO and diluting it with physiological saline or distilled water) or physiological saline was administered into the vein at a volume of 10 mL/kg over 4 minutes, and simultaneously the above-described micturition reflex operations were repeated until 30 minutes passed after initiation of the administration, while measuring the intravesical pressure and the volumes of micturition and residual urine. Also the frequency of uninhibited contraction occurrence during the above-described operations was recorded. The average values of the frequency of uninhibited contraction occurrence and the volume of residual urine in the experiment using several guinea pigs are shown in the following Table D.

TABLE D

| Compound | Dose (i.v., mg/kg) | Frequency of occurrence of uninhibited contraction (times/min) | | | Residual urine volume (mL) | | |
|---|---|---|---|---|---|---|---|
| | | before administration | after administration | volume change | before administration | after administration | volume change |
| Physiological saline | - | 1.10 | 1.08 | -0.02 | 1.44 | 1.43 | -0.01 |
| A-2 | 1 | 0.91 | 0.47 | -0.44 | 1.27 | 1.23 | -0.04 |
| | 10 | 0.9 | 0.78 | -0.13 | 1.27 | 1.02 | -0.25 |
| A-4 | 0.3 | 1.16 | 0.80 | -0.36 | 0.95 | 1.08 | +0.13 |
| | 3 | 1.16 | 0.49 | -0.67 | 0.95 | 1.11 | +0.16 |
| | 10 | 1.16 | 0.60 | -0.56 | 0.95 | 0.95 | 0.00 |
| A-7 | 1 | 0.96 | 0.79 | -0.17 | 0.82 | 0.86 | +0.04 |
| | 3 | 1.16 | 0.77 | -0.39 | 1.33 | 1.03 | -0.30 |
| | 10 | 1.11 | 0.64 | -0.47 | 1.06 | 0.64 | -0.42 |
| A-21 | 0.3 | 1.08 | 0.55 | -0.53 | 1.54 | 1.02 | -0.52 |
| | 3 | 1.44 | 0.78 | -0.66 | 1.37 | 0.88 | -0.49 |
| | 10 | 1.57 | 0.83 | -0.74 | 1.68 | 0.62 | -1.06 |
| A-26 | 0.3 | 1.12 | 1.02 | -0.10 | 1.02 | 0.91 | -0.11 |
| | 3 | 1.12 | 0.76 | -0.36 | 1.02 | 0.27 | -0.75 |
| | 10 | 1.12 | 0.59 | -0.53 | 1.02 | 0.48 | -0.54 |
| C-1 (methanesulfonate) | 0.1 | 1.20 | 1.82 | +0.62 | 0.25 | 0.13 | -0.12 |
| | 1 | 1.20 | 1.97 | +0.77 | 0.25 | 0.47 | 0.22 |
| | 3 | 1.20 | 0.68 | -0.52 | 0.25 | 0.21 | -0.04 |
| C-2 (methanesulfonate) | 0.3 | 2.09 | 1.45 | -0.64 | 0.43 | 0.11 | -0.32 |
| | 3 | 2.09 | 0.99 | -1.10 | 0.43 | 0.63 | +0.20 |
| | 10 | 0.57 | 0.00 | -0.57 | 0.37 | 0.03 | -0.34 |

(continued)

| Compound | Dose (i.v., mg/kg) | Frequency of occurrence of uninhibited contraction (times/min) | | | Residual urine volume (mL) | | |
|---|---|---|---|---|---|---|---|
| | | before administration | after administration | volume change | before administration | after administration | volume change |
| C-11 | 0.1 | 1.36 | 0.91 | -0.45 | 0.88 | 1.18 | +0.30 |
| | 1 | 1.36 | 1.03 | -0.33 | 0.88 | 1.02 | +0.14 |
| | 3 | 1.60 | 1.09 | -0.51 | 0.95 | 1.00 | +0.05 |

As shown in above Table D, the compounds used in the treating agent of the present invention also exhibited significant effect of reducing residual urine volume.

Thus the treating agents of this invention can be administered as PDE9 inhibitor or PDE9 inhibitor concurrently exhibiting slight PDE5-inhibiting activity, for therapy or treatment of PDE9-associated diseases of human and other mammals, orally or parenterally (e.g., intramuscular injection, intravenous injection, rectal administration, percutaneous administration and the like). When PDE5 is inhibited, urethra relaxation is induced, and hence the compounds which concurrently possess minor PDE5-inhibiting activity can be expected to also act to reduce residual urine volume.

The drugs of the present invention can be formulated, together with non-toxic excipients, any of the preparation forms such as solid (e.g., tablet, hard capsule, soft capsule, granule, powder, fine granule, pill, troche and the like); semi-solid (e.g., suppository, ointment and the like); or liquid (e.g., injection, emulsion, suspension, lotion, spray and the like). As non-toxic excipients useful for such formulations, for example, starch, gelatin, glucose, lactose, fructose, maltose, magnesium carbonate, talc, magnesium stearate, methyl cellulose, carboxymethyl cellulose or salts thereof, gum Arabic, polyethylene glycol, p-hydroxybenzoic acid alkyl ester, syrup, ethanol, propylene glycol, vaseline, Carbowax, glycerin, sodium chloride, sodium sulfite, sodium phosphate, citric acid and the like can be named. These drugs may also contain other therapeutically useful drugs.

Content of the compounds of the formula (I), (II), (III), (IV) or (V) in these drugs differs depending on such factors as the preparation form and administration route, while generally the compounds can be contained at a concentration of 0.1 - 50 wt% in solid and semi-solid forms, and of 0.05 - 10 wt%, in liquid form.

Doses of the compounds of the formula (I), (II), (III), (IV) or (V) are variable over broad ranges according to the kind of warm-blooded animals including human to be treated, kind of involved disease, administration route, seriousness of symptoms, doctor's diagnosis and so on. Whereas, generally they can be each within a range of 0.01 - 5 mg/kg, preferably 0.02 - 2 mg/kg, per day, it being obviously possible to administer doses less than the above lower limit or more than the above upper limit, according to the seriousness of individual patients' symptoms, doctor's diagnosis and so on, as aforesaid. Each dose can be administered single time per day or dividedly plural times per day.

## Examples

Hereinafter the present invention is more specifically explained, referring to Production Examples and working Examples.

### Production Example 1

5-Methyl-4-oxo-2-(thiophen-3-ylmethyl)-3,4-dihydrothieno[2,3-d]-pyrimidine-6-carboxylic acid (Compound No.: A-1)

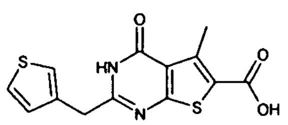

1-a): Synthesis of ethyl 5-methyl-4-oxo-2-(thiophen-3-ylmethyl)-3,4-dihydrothieno[2,3-d]pyrimidine-6-carboxylate

To 8 mL of 4N hydrogen chloride in dioxane solution, 515 mg of diethyl 5-amino-3-methylthiophene-2,4-dicarboxylate and 296 mg of 3-thiopheneacetonitrile were added, and stirred for 10 hours. Thereafter the liquid reaction mixture was poured on ice, and its pH was adjusted to 8-9 with 25% aqueous ammonia. Whereupon precipitated crystals were recovered by filtration and washed with water and hexane, by the order stated. The crude crystals were recrystallized from a liquid mixture of N,N-dimethylformamide and cyclohexane, to provide 397 mg of the title compound.
$^1$H-NMR(DMSO-$d_6$)δ:1.30(3H,t,J=7.1Hz),2.81(3H,s),3.97(2H,s), 4.30(2H,q,J=7.1Hz),7.0-7.6(3H,m),12.74(1H,br s)
MS(m/z):334($M^+$)

1-b): Synthesis of 5-methyl-4-oxo-2-(thiophen-3-ylmethyl)-3,4-dihydrothieno[2,3-d]pyrimidine-6-carboxylic acid

A mixture of 379 mg of the ethyl 5-methyl-4-oxo-2-(thiophen-3-ylmethyl)-3,4-dihydrothieno[2,3-d]pyrimidine-6-carboxylate which was synthesized in 1-a) above, 3.4 mL of 1N aqueous sodium hydroxide solution and 2.2 mL of ethanol was heated under reflux for 2 hours. The reaction liquid was allowed to cool off, poured on ice, and rendered

acidic with diluted hydrochloric acid. Thus precipitated crystals were recovered by filtration, washed with water and dried under reduced pressure by heating, to provide 320 mg of the title compound.
$^1$H-NMR(DMSO-$d_6$)δ2.79(3H,s),3.96(2H,s),7.0-7.6(3H,m), 12.69(1H,br s),13.32(1H,br s)
MS(m/z):306($M^+$)

Compounds of Production Examples 2-33 were synthesized in the manner similar to Production Example 1.

Production Example 2

5-Methyl-4-oxo-2-(thiophen-2-ylmethyl)-3,4-dihydrothieno-[2,3-d]pyrimidine-6-carboxylic acid (Compound No.: A-2)

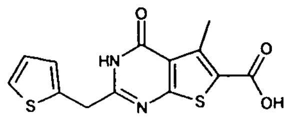

$^1$H-NMR(DMSO-$d_6$)δ:2.79(3H,s),4.17(2H,s),6.9-7.5(3H,m), 12.75(1H,br s),13.35(1H,br s)
MS(m/z):306($M^+$)

Production Example 3

2-(5-Chlorothiophen-2-ylmethyl)-5-methyl-4-oxo-3,4-dihydrothieno[2,3-d]pyrimidine-6-carboxylic acid (Compound No.: A-3)

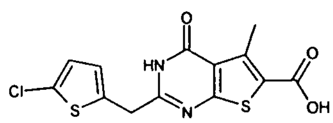

$^1$H-NMR(DMSO-$d_6$)δ:2.79(3H,s),4.13(2H,s),6.91(1H,d,J=3.9Hz), 6.98(1H,d,J=3.9Hz),12.74(1H,br s),13.37(1H,br s)
MS(m/z):342($M^+$+2),340($M^+$)

Production Example 4-1)

2-(3-Chlorobenzyl)-5-methyl-4-oxo-3,4-dihydrothieno[2,3-d]-pyrimidine-6-carboxylic acid (Compound No.: A-4)

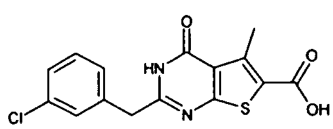

$^1$H-NMR(DMSO-$d_6$)δ:2.79(3H,s),3.98(2H,s),7.2-7.4(3H,m), 7.45(1H,s),12.72(1H,br s),13.33(1H,br s)
MS(m/z):336($M^+$+2),334($M^+$)

Production Example 4-2)

2-(3-Chlorobenzyl)-5-methyl-4-oxo-3,4-dihydrothieno[2,3-d]-pyrimidine-6-carboxylic acid sodium salt

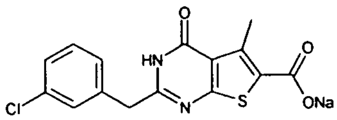

The sodium salt of the compound of above Production Example 4-1) was obtained.
$^1$H-NMR(DMSO-$d_6$)δ:2.73(3H,s),3.92(2H,s),7.2-7.4(3H,m), 7.44(1H,s),12.34(1H,br s)

Production Example 4-3)

2-(3-Chlorobenzyl)-5-methyl-4-oxo-3,4-dihydrothieno[2,3-d]-Pyrimidine-6-carboxylic acid sodium salt • 1/2 ethanolate

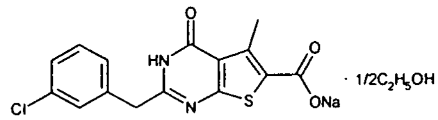

A sodium salt · 1/2 ethanolate of the compound of Production Example 4-1) was obtained.
$^1$H-NMR(DMSO-$d_6$)δ:1.06(1.5H,t,J=7.0Hz),2.73(3H,s), 3.44(1H,q,J=6.9Hz),3.92(2H,s),4.34(0.5H,br s),7.2-7.4(3H,m), 7.44(1H,s),12.32(1H,br s) (The underlined parts correspond to the ethanol-derived peaks.)

Production Example 5

2-(3-Bromobenzyl)-5-methyl-4-oxo-3,4-dihydrothieno[2,3-d]-pyrimidine-6-carboxylic acid (Compound No.: A-5)

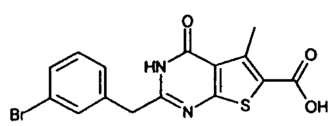

$^1$H-NMR(DMSO-$d_6$)δ:2.79(3H,s),3.97(2H,s),7.2-7.7(4H,m), 12.71(1H,br s),13.34(1H,br s)
MS(m/z):380($M^+$+2), 378($M^+$)

Production Example 6

5-Methyl-4-oxo-2-(3-trifluoromethylbenzyl)-3,4-dihydrothieno[2,3-d]pyrimidine-6-carboxylic acid (Compound No.: A-6)

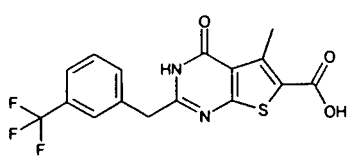

$^1$H-NMR(DMSO-$d_6$)δ:2.79(3H,s),4.08(2H,s),7.5-7.7(3H,m), 7.76(1H,s),12.75(1H,br s),13.34(1H,br s)
MS(m/z):368($M^+$)

Production Example 7-1)

2-(3,4-Dichlorobenzyl)-5-methyl-4-oxo-3,4-dihydrothieno-[2,3-d]pyrimidine-6-carboxylic acid (Compound No.: A-7)

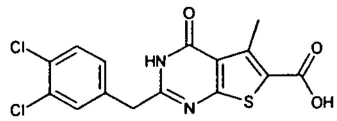

$^1$H-NMR(DMSO-$d_6$)δ:2.79(3H,s),3.99(2H,s),7.3-7.7(3H,m), 12.71(1H,br s),13.33(1H,br s)
MS(m/z):370($M^+$+2),368($M^+$)

Production Example 7-2)

2-(3,4-Dichlorobenzyl)-5-methyl-4-oxo-3,4-dihydrothieno-[2,3-d]pyrimidine-6-carboxylic acid sodium salt

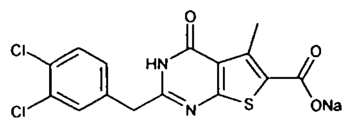

The sodium salt of the compound of Production Example 7-2) was obtained.
$^1$H-NMR(DMSO-$d_6$)δ:2.73(3H,s),3.93(2H,s),7.34(1H,d'd,J=1.9,8.5Hz), 7.59(1H,d,J=8.5Hz),7.64(1H,d,J=1.9Hz),12.31 (1H,br s)

Production Example 7-3)

2-(3,4-Dichlorobenzyl)-5-methyl-4-oxo-3,4-dihydrothieno-[2,3-d]pyrimidine-6-carboxylic acid sodium salt • 1/2 ethanolate

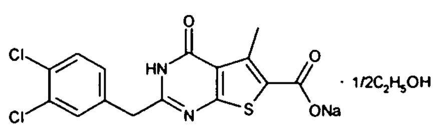

The sodium salt · 1/2 ethanolate of the compound of Production Example 7-3) was obtained.
$^1$H-NMR(DMSO-$d_6$)δ:1.06(1.5H,t,J=7.0Hz),2.73(3H,s), 3.4-3.5(1H,m),3.93(2H,s),4.34(0.5H,br t),7.34(1H,dd, J=1.9,8.5Hz), 7.59(1H,d,J=8.5Hz),7.64(1H,d,J=1.9Hz),12.31(1H,br s) (The underlined parts correspond to the ethanol-derived peaks.)

Production Example 8

5-Methyl-4-oxo-2-(2-trifluoromethylthiobenzyl)-3,4-dihydrothieno[2,3-d]pyrimidine-6 -carboxylic acid (Compound No.: A-8)

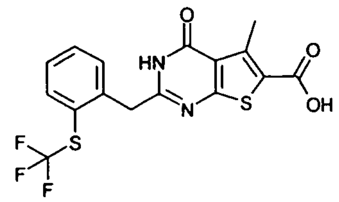

$^1$H-NMR(DMSO-$d_6$)δ:2.80(3H,s),4.32(2H,s),7.4-7.6(3H,m), 7.7-7.8(1H,m),12.77(1H,br s),13.32(1H,br s)
MS(m/z):400($M^+$)

Production Example 9

2-(4-Fluoro-3-trifluoromethylbenzyl)-5-methyl-4-oxo-3,4-dihydrothieno[2,3-d]pyrimidine-6-carboxylic acid (Compound No.: A-9)

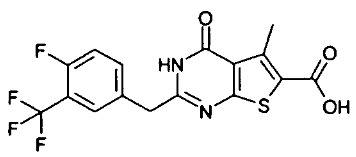

$^1$H-NMR(DMSO-$d_6$)δ:2.79(3H,s),4.07(2H,s),7.4-7.5(1H,m), 7.6-7.8(1H,m),7.8-7.9(1H,m),12.73(1H,br s),13.34(1H,br s)
MS(m/z):386($M^+$)

Production Example 10

2-(3-Chloro-4-fluorobenzyl)-5-methyl-4-oxo-3,4-dihydrothieno-[2,3-d]pyrimidine-6-carboxylic acid (Compound No.: A-10)

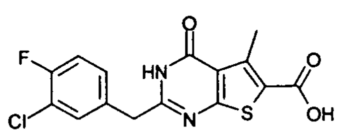

$^1$H-NMR(DMSO-$d_6$)δ:2.79(3H,s),3.97(2H,s),7.3-7.5(2H,m), 7.5-7.7(1H,m),12.69(1H,br s),13.34(1H,br s)
MS(m/z):354($M^+$+2),352($M^+$),183(base)

Production Example 11

2-(5-Chloro-2-fluorobenzyl)-5-methyl-4-oxo-3,4-dihydrothieno-[2,3-d]pyrimidine-6-carboxylic acid (Compound No.: A-11)

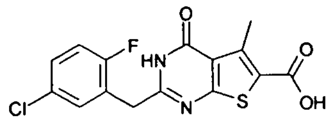

H-NMR(DMSO-$d_6$)s:2.80(3H,s),4.05(2H,s),7.26(1H,t,J=9.1Hz), 7.3-7.5(1H,m),7.52(1H,dd,J=2.7,6.2Hz),12.73(1H,br s), 13.36(1H,br s)
MS(m/z):354($M^+$+2), 352($M^+$,base)

Production Example 12

2-(2,5-Difluorobenzyl)-5-methyl-4-oxo-3,4-dihydrothieno-[2,3-d]pyrimidine-6-carboxylic acid (Compound No.: A-12)

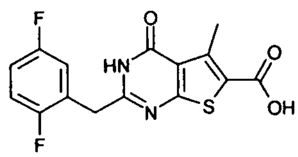

$^1$H-NMR(DMSO-$d_6$)δ2.80(3H,s),4.06(2H,s),7.1-7.4(3H,m), 12.79(1H,br s),13.34(1H,br s)
MS(m/z):336($M^+$)

Production Example 13

2-(Cyclopent-1-enylmethyl)-5-methyl-4-oxo-3,4-dihydrothieno-[2,3-d]pyrimidine-6-carboxylic acid (Compound No.: A-13)

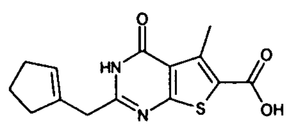

$^1$H-NMR(DMSO-$d_6$)δ:1.7-1.9(2H,m),2.2-2.4(4H,m),2.80(3H,s), 3.41(2H,s),5.48(1H,s),12.51(1H,br s),13.31(1H,br s)
MS(m/z):290($M^+$)

Production Example 14

2-(Cyclohex-1-enylmethyl)-5-methyl-4-oxo-3 4-dihydrothieno-[2,3-d]pyrimidine-6-carboxylic acid (Compound No.: A-14)

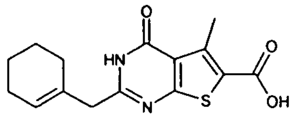

$^1$H-NMR(DMSO-$d_6$)δ:1.4-1.5(2H,m),1.5-1.6(2H,m),1.9-2.0(4H,m), 2.77(3H,s),3.23(2H,s),5.52(1H,s),12.42(1H,s)
MS(m/z):304(M$^+$),262(base)

Production Example 15

4-Oxo-2-(thiophen-3-ylmethyl)-3,4-dihydrothieno-[2,3-d]pyrimidine-6-carboxylic acid (Compound No.: A-15)

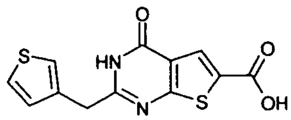

$^1$H-NMR(DMSO-$d_6$)δ:4.00(2H,s),7.10(1H,dd,J=1.5,5.0Hz), 7.3-7.4(1H,m),7.49(1H,dd,J=3.0,5.0Hz),7.83(1H,s),12.83 (1H,s), 13.52(1H,br s)
MS(m/z):292(M$^+$)

Production Example 16

4-Oxo-2-(thiophen-2-ylmethyl)-3,4-dihydrothieno[2,3-d]-pyrimidine-6-carboxylic acid (Compound No.: A-16)

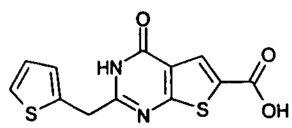

$^1$H-NMR(DMSO-$d_6$)δ:4.20(2H,s),6.99(1H,dd,J=3.5,5.3Hz), 7.0-7.1(1H,m),7.42(1H,dd,J=1.2,5.0Hz),7.85(1H,s),12.89 (1H,s), 13.57(1H,br s)
MS(m/z):292(M$^+$)

Production Example 17

2-Benzyl-4-oxo-3,4-dihydrothieno[2,3-d]pyrimidine-6-carboxylic acid (Compound No.: A-17)

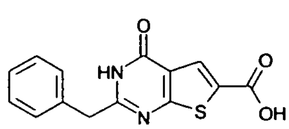

The title compound was synthesized from ethyl 2-benzyl-4-oxo-3,4-dihydrothieno[2,3-d]pyrimidine-6-carboxylate as synthesized in Production Example 45, in the manner similar to Example 1.
$^1$H-NMR(DMSO-$d_6$)δ:3.99(2H,s),7.2-7.4(5H,m),7.84(1H,s), 12.87(1H,br s),13.56(1H,br s)
MS(m/z):286($M^+$),169(base)

Production Example 18

2-(3-Chlorobenzyl)-4-oxo-3,4-dihydrothieno[2,3-d]-pyrimidine-6-carboxylic acid (Compound No.: A-18)

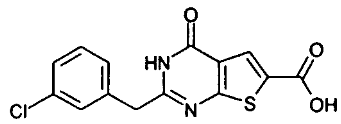

$^1$H-NMR(DMSO-$d_6$)δ:4.01(2H,s),7.3-7.4(3H,m),7.4-7.5(1H,m), 7.84(1H,s),12.87(1H,s),13.50(1H,br s)
MS(m/z):320($M^+$)

Production Example 19

4-Oxo-2-(3-trifluoromethylbenzyl)-3,4-dihydrothieno-[2,3-d]pyrimidine-6-carboxylic acid (Compound No.: A-19)

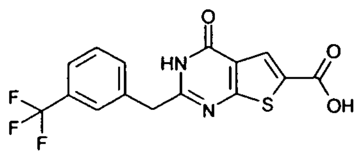

$^1$H-NMR(DMSO-$d_6$)δ:4.11(2H,s),7.5-7.7(3H,m),7.77(1H,s), 7.84(1H,s),12.89(1H,s),13.58(1H,br s)
MS(m/z):354($M^+$)

Production Example 20

2-(3,4-Dichlorobenzyl)-4-oxo-3,4-dihydrothieno[2,3-d]-pyrimidine-6-carboxylic acid (Compound No.: A-20)

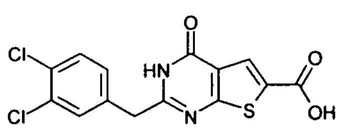

$^1$H-NMR(DMSO-$d_6$)δ:4.02(2H,s),7.36(1H,dd,J=1.9,8.3Hz), 7.60(1H,d,J=8.3Hz),7.66(1H,d,J=1.9Hz),7.85(1H,s), 12.85 (1H,br s),13.57(1H,br s)
MS(m/z):356($M^+$+2), 354($M^+$),169(base)

Production Example 21

2-(Cyclopent-1-enylmethyl)-4-oxo-3,4-dihydrothieno[2,3-d]-pyrimidine-6-carboxylic acid (Compound No.: A-21)

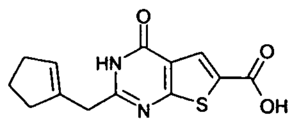

$^1$H-NMR(DMSO-$d_6$)δ:1.3-1.4(2H,m),2.2.2.4(4H,m),3.44(2H,s), 5.4-5.5(1H,m),7.85(1H,s);12.66(1H,s),13.55(1H,br s)
MS(m/z):276($M^+$)

Production Example 22

5-Methyl-2-(thiophen-3-ylmethyl)-4-thioxo-3,4-dihydrothieno-[2,3-d]pyrimidine-6-carboxylic acid (Compound No.: A-22)

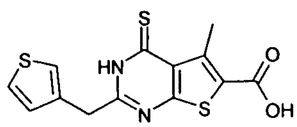

$^1$H-NMR(DMSO-$d_6$)δ:3.05(3H,s),4.10(2H,s),7.10(1H,dd,J=1.2,5.0Hz), 7.3-7.4(1H,m),7.4-7.6(1H,m),13.57(1H,br s), 13.94(1H,br s)
MS(m/z):322($M^+$,base)

Production Example 23

5-Methyl-2-(thiophen-2-ylmethyl)-4-thioxo-3,4-dihydrothieno-[2,3-d]pyrimidine-6-carboxylic acid (Compound No.: A-23)

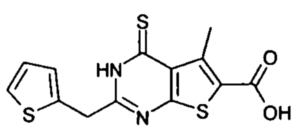

$^1$H-NMR(DMSO-$d_6$)δ:3.05(3H,s),4.31(2H,s),6.99(1H,dd,J=3.5,5.0Hz), 7.05(1H,dd,J=1.3,3.5Hz),7.43(1H,dd, J=1.3,5.0Hz), 13.59(1H,br s),14.00(1H,br s)
MS(m/z):322($M^+$),97(base)

Production Example 24

2-Benzyl-5-methyl-4-thioxo-3,4-dihydrothieno[2,3-d]-pyrimidine-6-carboxylic acid (Compound No.: A-24)

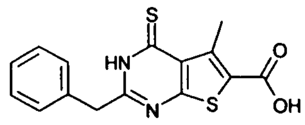

$^1$H-NMR(DMSO-$d_6$)δ:3.05(3H,s),4.10(2H,s),7.2-7.4(5H,m), 13.56(1H,br s),13.98(1H,br s)
MS(m/z):316($M^+$,base)

Production Example 25

2-(3-Bromobenzyl)-5-methyl-4-thioxo-3,4-dihydrothieno-[2,3-d]pyrimidine-6-carboxylic acid (Compound No.: A-25)

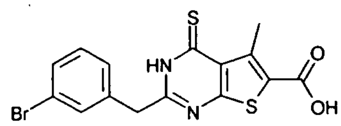

$^1$H-NMR(DMSO-$d_6$)δ:3.05(3H,s),4.11(2H,s),7.2-7.4(2H,m), 7.4-7.5(1H,m),7.5-7.7(1H,m),13.58(1H,br s),13.97(1H,br s)
MS(m/z):396($M^+$+2,base),394($M^+$)

Production Example 26

2-(3-Chlorobenzyl)-5-methyl-4-thioxo-3,4-dihydrothieno-[2,3-d]pyrimidine-6-carboxylic acid (Compound No.: A-26)

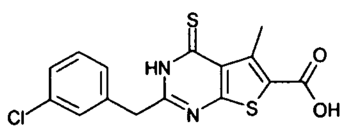

$^1$H-NMR(DMSO-$d_6$)δ:3.05(3H,s),4.12(2H,s),7.2-7.5(4H,m), 13.57(1H,br s),13.97(1H,br s)
MS(m/z):352($M^+$+2), 350($M^+$,base)

Production Example 27

5-Methyl-4-thioxo-2-(3-trifluoromethylbenzyl)-3,4-dihydrothieno[2,3-d]pyrimidine-6-carboxylic acid (Compound No.: A-27)

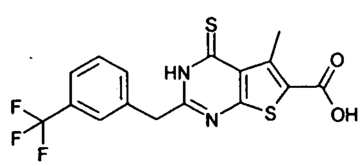

$^1$H-NMR(DMSO-$d_6$)δ:3.05(3H,s),4.22(2H,s),7.5-7.7(3H,m), 7.78(1H,s),13.58(1H,br s),14.00(1H,br s)
MS(m/z):384($M^+$,base)

Production Example 28

2-(3,4-Dichlorobenzyl)-5-methyl-4-thioxo-3,4-dihydrothieno-[2,3-d]pyrimidine-6-carboxylic acid (Compound No.: A-28)

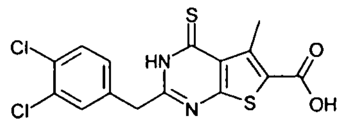

$^1$H-NMR(DMSO-$d_6$)δ:3.05(3H,s),4.13(2H,s),7.35(1H,dd,J=1.9,8.3Hz), 7.60(1H,d,J=8.3Hz),7.67(1H,d,J=1.9Hz),13.57 (1H,br),14.96(1H,br s)
MS(m/z):386 ($M^+$+2),384($M^+$,base)

Production Example 29

2-(3-Chloro-4-fluorobenzyl)-5-methyl-4-thioxo-3,4-dihydrothieno[2,3-d]pyrimidine-6-carboxylic acid (Compound No.: A-29)

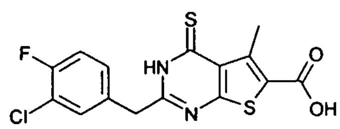

$^1$H-NMR(DMSO-$d_6$)δ:3.05(3H,s),4.11(2H,s),7.3-7.5(2H,m), 7.5-7.7(1H,m),13.59(1H,br s),13.97(1H,br s)
MS(m/z):370($M^+$+2), 368($M^+$,base)

Production Example 30

2-(Cyclohex-l-enylmethyl)-5-methyl-4-thioxo-3,4-dihydrothieno[2,3-d]pyrimidine-6-carboxylic acid (Compound No.: A-30)

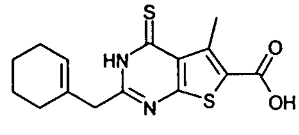

$^1$H-NMR(DMSO-$d_6$)δ:1.4-1.7(4H,m),1.8-2.1(4H,m),3.06(3H,s), 3.39(2H,s),5.53(1H,s),13.56(1H,br s),13.72(1H,br s)
MS(m/z):320($M^+$,base)

Production Example 31

2-(Cyclopent-1-enylmethyl)-5-methyl-4-thioxo-3,4-dihydrothieno[2,3-d]pyrimidine-6-carboxylic acid and 2-cyclopentylidenemethyl-5-methyl-4-thioxo-3,4-dihydrothieno-[2,3-d]pyrimidine-6-carboxylic acid (Compound No.: A-31)

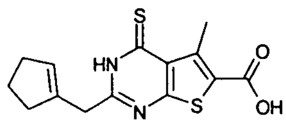
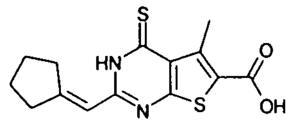

cyclopent-1-enylmethyl form
$^1$H-NMR(DMSO-$d_6$)δ:1.7-1.9(2H,m),2.2-2.4(4H,m),3.06(3H,s), 3.54(2H,s),5.4-5.5(1H,m),13.47(1H,br s),13.77(1H,br s)
cyclopentylidenemethyl form
$^1$H-NMR(DMSO-$d_6$)δ:1.6-1.9(4H,m),2.4-2.7(2H,m),2.8-2.9(2H,m), 3.06(3H,s),6.4-6.5(1H,m),13.47(1H,br s),13.77(1H, br s) MS(m/z):314($M^+$)

Production Example 32

2-Benzyl-4-thioxo-3,4-dihydrothieno[2,3-d]pyrimidine-6-carboxylic acid (Compound No.: A-32)

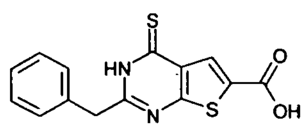

$^1$H-NMR(DMSO-$d_6$)δ:4.13(2H,s),7.2-7.4(5H,m),8.00(1H,s), 13.77(1H,br s),14.26(1H,br s)
MS(m/z):302($M^+$,base)

Production Example 33

2-(3,4-Dichlorobenzyl)-4-thioxo-3,4-dihydrothieno[2,3-d]-pyrimidine-6-carboxylic acid (Compound No.: A-33)

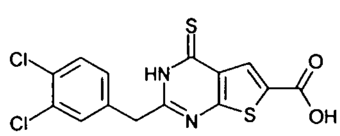

$^1$H-NMR(DMSO-$d_6$)δ:4.16(2H,s),7.35(1H,dd,J=1.9,8.3Hz), 7.61(1H,d,J=8.3Hz),7.67(1H,d,J=1.9Hz),8.01(1H,s), 13.77 (1H,br s),14.22(1H,br s)
MS(m/z):372($M^+$+2),370($M^+$,base)

Production Example 34

2-(3-Chlorobenzyl)-4-oxo-3,4-dihydroquinazoline-7-carboxylic acid (Compound No.: B-1)

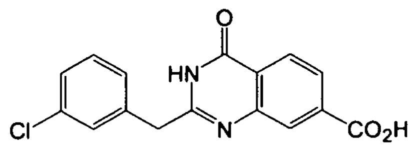

34-a): Synthesis of methyl 2-(3-chlorobenzyl)-4-oxo-3,4-dihydroquinazoline-7-carboxylate

A mixture of 628 mg of dimethyl aminoterephthalate, 546 mg of 3-chlorophenylacetonitrile and 15 mL of 4N hydrogen chloride in dioxane solution was stirred for 7 hours at room temperature. Further continuing the stirring for 63 hours at 30°C and 25 hours at 70°C, ice was added to the reaction liquid. Then 7 mL of 25% aqueous ammonia was added, and the precipitated crystals were recovered by filtration. The crystals were washed successively with water, ether and chloroform by the order stated. Drying the same in flowing air under heating, 670 mg of the title compound was obtained.
$^1$H-NMR(DMSO-$d_6$,δ):3.91(3H,s),3.99(2H,s),7.3-7.4(3H,m), 7.4-7.5(1H,m),7.96(1H,dd,J=1.4,8.3Hz),8.08(1H,d, J=1.4Hz), 8.19(1H,d,J=8.3Hz),12.61(1H,br s)
MS(m/z):327($M^+$-1)

34-b): Synthesis of 2-(3-chlorobenzyl)-4-oxo-3,4-dihydroquinazoline-7-carboxylic acid

A mixture of 336 mg of the methyl 2-(3-chlorobenzyl)-4-oxo-3,4-dihydroquinazoline-7-carboxylate as synthesized in above 34-a), 3.0 mL of 1N aqueous sodium hydroxide solution and 6 mL of ethanol was heated under reflux for 2.5 hours. The reaction liquid was allowed to cool off, and to which 3.0 mL of 1N hydrochloric acid and 5 mL of water were added. The precipitated crystals were recovered by filtration, washed with water and dried in flowing air under heating, to provide 300 mg of the title compound.
$^1$H-NMR(DMSO-$d_6$,δ)13.99(2H,s),7.3-7.4(3H,m),7.4-7.5(1H,m), 7.95(1H,dd,J=1.4,8.3Hz),8.07(1H,d,J=1.4Hz),8.17 (1H,d,J=8.3Hz), 12.58(1H,br s),13.40(1H,br s)
MS(m/z):313($M^+$-1)

In the manner similar to Production Example 34, the compounds of Production Examples 35-42 were synthesized.

Production Example 35

2-(3,4-Dichlorobenzyl)-4-oxo-3,4-dihydroquinazoline-7-carboxylic acid (Compound No.: B-2)

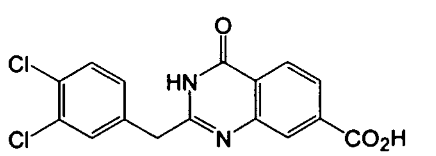

$^1$H-NMR(DMSO-$d_6$,δ):3.99(2H,s),7.38(1H,dd,J=2.4,8.3Hz), 7.59(1H,d,J=8.3Hz),7.68(1H,d,J=2.0Hz),7.9-8.0(1H,m), 8.05(1H,d,J=1.5Hz),8.17(1H,d,J=8.3Hz),12.56(1H,br s), 13.41(1H,br s)
MS(m/z):349($M^+$+2),347($M^+$,base)

Production Example 36

2-(3,4-Dimethoxybenzyl)-4-oxo-3.4-dihydroquinazoline-7-carboxylic acid (Compound No.: B-3)

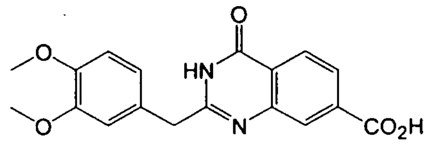

The title compound was obtained from the methyl 2-(3,4-dimethoxybenzyl)-4-oxo-3,4-dihydroquinazoline-7-carboxylate as synthesized in Production Example 3, in the manner similar to Example 2.
$^1$H-NMR(DMSO-$d_6$,s):3.70(3H,s),3.74(3H,s),3.86(2H,s), 6.8-7.0(2H,m),7.04(1H,s),7.9-8.0(1H,m),8.06(1H,d,J=1.1Hz), 8.09(1H,d,J=8.3Hz),12.43(1H,br s)
MS(m/z):340($M^+$,base)

Production Example 37

2-(2-Methoxybenzyl)-4-oxo-3,4-dihydroquinazoline-7-carboxylic acid (Compound No.: B-4)

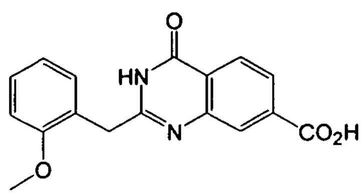

$^1$H-NMR(DMSO-ds,8):3.75(3H,s),3.93(2H,s),6.89(1H,dt,J=0.8,7.4Hz), 6.98(1H,d,J=8.1Hz),7.1-7.2(1H,m),7.2-7.3(1H, m), 7.91(1H,dd,J=1.6,8.1Hz),7.97(1H,d,J=1.2Hz),8.17(1H,d,J=8.1Hz), 12.42(1H,br s),13.34(1H,br s)
MS(m/z):310($M^+$),279(base)

Production Example 38

2-(3-Methoxybenzyl)-4-oxo-3,4-dihydroquinazoline-7-carboxylic acid (Compound No.: B-5)

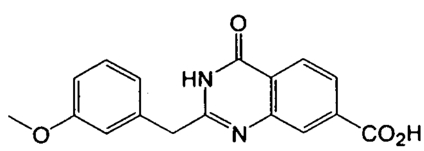

$^1$H-NMR(DMSO-$d_6$,$\delta$):3.72(3H,s),3.90(2H,s),6,8-6.9(1H,m), 6.94(1H,d,J=7.7Hz),6.9-7.0(1H,m),7.22(1H,t,J=7.9Hz), 7.92(1H,dd,J=1.5,8.3Hz),8.07(1H,d,J=1.5Hz),8.15(1H,d,J=8.3Hz), 12.53(1H,br s),13.37(1H,br s)
MS(m/z):310($M^+$), 309(base)

Production Example 39

4-Oxo-2-(2-pyridylmethyl)-3,4-dihydroquinazoline-7-carboxylic acid (Compound No.: B-6)

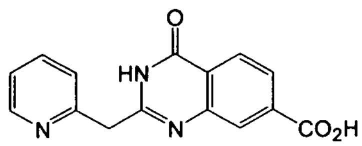

MS(m/z):281($M^+$),280(base)

Production Example 40

4-Oxo-2-(3-pyridylmethyl)-3,4-dihydroquinazoline-7-carboxylic acid (Compound No.: B-7)

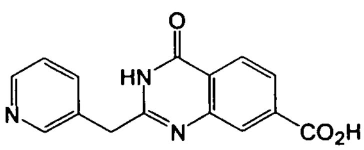

MS(m/z):281 ($M^+$), 280(base)

Production Example 41

4-Oxo-2-(2-thienylmethyl)-3,4-dihydroquinazoline-7-carboxylic acid (Compound No.: B-8)

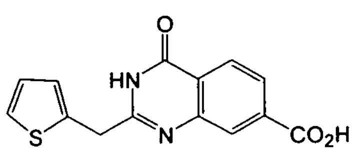

$^1$H-NMR(DMSO-$d_6$,δ):4.15(2H,s),6.9-7.0(1H,m),7.0-7.1(1H,m), 7.3-7.4(1H,m),7.9-8.0(1H,m),8.08(1H,d,J=1.1Hz), 8.18 (1H,d,J=8.3Hz),12.57(1H,br s),13.38(1H,br s)
MS(m/z):286($M^+$,base)

Production Example 42

2-(3-Chlorobenzyl)-3-methyl-4-oxo-3,4-dihydroguinazoline-7-carboxylic acid (Compound No.: B-9)

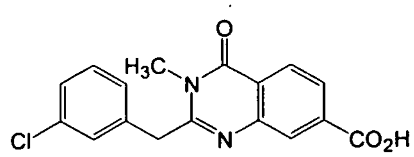

[1]H.NMR(DMSO-$d_6$,δ)13.51(3H,s),4.34(2H,s),7.2-7.5(4H,m), 7.98(1H,dd,J=1.4,8.5Hz),8.05(1H,d,J=1.4Hz),8.22(1H,d, J=8.5Hz), 13.43(1H,br s)
MS(m/z):327 ($M^+$-1,base)

Production Example 43

7-Chloro-1-isopropylimidazo[1,5-a]quinoxalin-4(5H)-one (Compound No.: C-1)

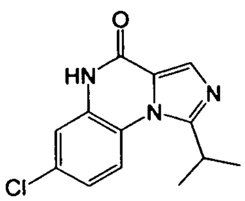

43-a): Synthesis of 1-(4-chloro-2-nitrophenyl)-2-isopropyl-1H-imidazole

A mixture of 1.01 g of 4-chloro-1-fluoro-2-nitrobenzene, 634 mg of 2-isopropylimidazole, 1.46 mL of N,N-diisopropylethylamine and 12 mL of acetonitrile was heated under reflux for 15 hours. The solvent was distilled off, water was added, and the reaction liquid was rendered acidic with diluted hydrochloric acid, followed by washing with diethyl ether. The aqueous layer was rendered alkaline with aqueous sodium hydroxide solution, and extracted twice with chloroform. The organic layer was washed with water, dried over magnesium sulfate, and removed of the solvent by distillation to provide 0.98 g of the title compound.
$^1$H-NMR(CDCls,δ):1.22(6H,d,J=6.9Hz),2.5-2.8(1H,m), 6.81(1H,d,J=1.2Hz),7.09(1H,d,J=1.5Hz),7.39(1H,d,J=8.5Hz), 7.71(1H,dd,J=2.3,8.5Hz),8.05(1H,d,J=2.7Hz)
MS(m/z):267($M^+$+2), 265(M+)

43-b): Synthesis of 5-chloro-2-(2-isopropyl-1H-imidazol-1-yl)aniline

A mixture of 0.98 g of the 1-(4-chloro-2-nitrophenyl)-2-isopropyl-1H-imidazole as synthesized in above 43-a), 4.16 g of tin (II) chloride dihydrate and 8.8 mL of ethanol was heated under reflux for 2.7 hours. Neutralizing the reaction liquid with 2N aqueous sodium hydroxide solution under cooling with ice, chloroform was added, followed by filtration through Celite and washing with chloroform. The filtrate was transferred into a separating funnel and extracted twice with chloroform. The organic layer was washed with water, dried over magnesium sulfate, and removed of the solvent by distillation to provide 0.72 g of the title compound.
$^1$H-NMR($CDCl_3$,δ):1.21(6H,d,J=4.2Hz),2.6-2.9(1H,m),3.63(2H,br s) 6.7-6.9(3H,m),6.99(1H,d,J=8.5Hz), 7.12(1H,d, J=1.5Hz)
MS(m/z):237($M+^{+2}$), 235(M+)

43-c): Synthesis of 7-chloro-1-isopropylimidazo[1,5-a]guinoxalin-4(5H)-one

A mixture of 720 mg of the 5-chloro-2-(2-isopropyl-1H-imidazol-1-yl)aniline as synthesized in above 43-b), 743 mg of 1,1'-carbonyldiimidazole and 23 mL of 1,2-dichlorobenzene was heated under reflux for 4.3 hours in nitrogen atmosphere. The reaction liquid was allowed to cool off and the precipitated crystals were recovered by filtration. The crystals were washed with methanol and then dried by heating under reduced pressure to provide 600 mg of the title

compound.
$^1$H-NMR(DMSO-$d_6$,δ):1.39(6H,d,J=6.6Hz),3.7-3.9(1H,m), 7.28(1H,dd,J=2.7,8.9Hz),7.35(1H,d,J=2.3Hz),7.79(1H,s), 8.05(1H,d,J=8.9Hz),11.44(1H,br s)
MS(m/z):263($M^+$+2),261($M^+$)

In the manner similar to Production Example 43, the compounds of Production Examples 44-52 were synthesized.

Production Example 44

1-Isopropyl-N,N-dimethyl-4-oxo-4,5-dihydroimidazo[1,5-a]-quinoxaline-7-carboxamide (Compound No.: C-2)

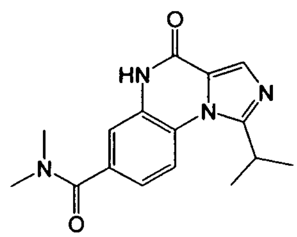

$^1$H-NMR(DMSO-$d_6$,δ):1.42(6H,d,J=6.6Hz),2.8-3.1(6H,m), 3.7-3.9(1H,m),7.29(1H,dd,J=1.9,8.5Hz),7.35(1H,d, J=1.5Hz), 7.80(1H,s),8.08(1H,d,J=8.5Hz),11.44(1H,br s)
MS(m/z):298($M^+$)

Production Example 45

1-Cyclohexyl-8-methoxy-N,N-dimethyl-4-oxo-4,5-dihydroimidazo[1,5-a]quinoxaline-7-carboxamide (Compound No.: C-3)

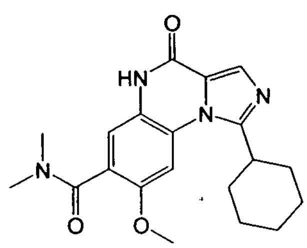

$^1$H-NMR(DMSO-$d_6$,δ)=1.2-1.4(1H,m),1.4-1.6(2H,m),1.6-2.0(5H,m), 2.17(2H,br d,J=12.7Hz),2.80(3H,s),2.99(3H,s), 3.4-3.6(1H,m), 3.94(3H,s),7.13(1H,s),7.51(1H,s),7.78(1H,s),11.31(1H,br s)
MS(m/z):368($M^+$)

Production Example 46

1-Cyclohexyl-8-ethoxy-N,N-dimethyl-4,5-dihydroimidazo-[1,5-a]quinoxaline-7-carboxamide (Compound No.: C-4)

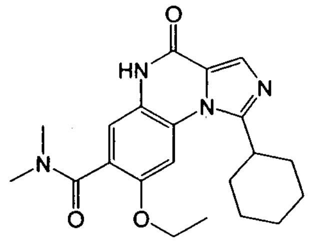

$^1$H-NMR(DMSO-$d_6$,δ):1.2-1.6(3H,m),1.38(3H,t,J=6.9Hz), 1.6-1.9(3H,m),1.87(2H,br d,J=13.1Hz),2.15(2H,br d, J=12.0Hz), 2.82(3H,s),3.00(3H,s),3.4-3.6(1H,m),4.21(2H,q,J=6.8Hz),7.14(1H,s), 7.48(1H,s),7.77(1H,s),11.30(1H,br s)
MS(m/z):382($M^+$)

Production Example 47

8-Chloro-1-cyclohexyl-N,N-dimethyl-4-oxo-4,5-dihydroimidazo-[1,5-a]quinoxaline-7-carboxamide (Compound No.: C-5)

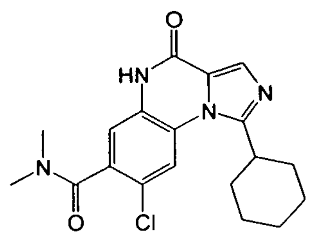

$^1$H-NMR(DMSO-$d_6$,δ):1.2-1.4(1H,m),1.4-1.6(2H,m),1.6-1.9(3H,m), 1.86(2H,br d,J=13.1Hz),2.09(2H,br d,J=13.1Hz), 2.83(3H,s),3.03(3H,s),3.3-3.5(1H,m),7.21(1H,s),7.81(1H,s), 7.89(1H,s),11.56(1H,br s)
MS(m/z):374($M^+$+2), 372($M^+$)

Production Example 48

1-[(8-Chloro-l-cyclohexyl-4-oxo-4,5-dihydroimidazo[1,5-a]-quinoxalin-7-yl)carbonyl]-4-(2-hydroxyethyl)piperazine (Compound No.: C-6)

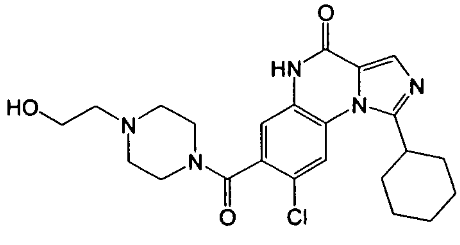

$^1$H-NMR(DMSO-$d_6$,δ):1.2-1.4(1H,m),1.4-1.9(5H,m), 1.86(2H,br d,J=12.7Hz),2.09(2H,br d,J=12.7Hz),2.3-2.5(4H,m), 3.1-3.3(2H,m),3.3-3.6(3H,m),3.66(2H,br s),4.4-4.5(1H,m), 7.22(1H,s),7.81(1H,s),7.88(1H,s),11.53(1H,br s)

MS(m/z):457($M^+$)

Production Example 49

3-(7-Ethyl-4-oxo-4 5-dihydroimidazo[1,5-a]quinoxalin-1-yl)-propionic acid (Compound No.: C-7)

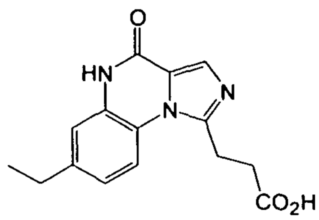

$^1$H-NMR(DMSO-$d_6$,δ):1.21(3H,t,J=7.5Hz),2.66(2H,q,J=7.5Hz), 2.91(2H,t,J=6.7Hz),3.46(2H,t,J=6.7Hz),7.12(1H,dd, J=1.9, 8.9Hz), 7.18(1H,d,J=1.9Hz),7.74(1H,s),7.96(1H,d,J=8.9Hz),11.29(1H,br s), 12.20(1H,br s)
MS(m/z):285($M^+$),240(base)

Production Example 50

3-(4-Oxo-7-trifluoromethyl-4,5-dihydroimidazo[1,5-a]-quinoxalin-1-yl)propionic acid (Compound No.: C-8)

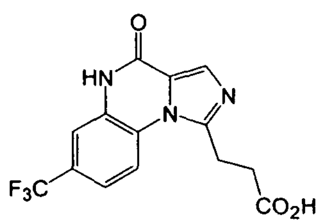

$^1$H-NMR(DMSO-$d_6$,δ):2.93(2H,t,J=6.7Hz),3.50(2H,t,J=6.7Hz), 7.60(1H,dd,J=1.5,8.9Hz),7.65(1H,d,J=1.5Hz),7.82(1H, s), 8.25(1H,d,J=8.9Hz),11.57(1H,br s),12.24(1H,br s)
MS(m/z):325($M^+$),280(base)

Production Example 51

3-(7-Bromo-4-oxo-4,5-dihydroimidazo[1,5-a]quinoxalin-1-yl)-propionic acid (Compound No.: C-9)

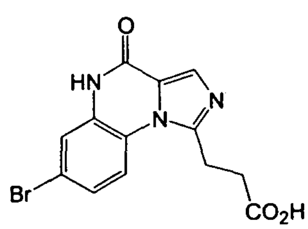

$^1$H-NMR(DMSO-$d_6$,δ):2.90(2H,t,J=6.6Hz),3.44(2H,t,J=6.6Hz), 7.42(1H,dd,J=2.3,9.1Hz),7.49(1H,d,J=2.3Hz),7.78(1H,s), 7.99(1H,d,J=9.1Hz),11.43(1H,s),12.22(1H,br s)
MS(m/z):337($M^+$+2),335($M^+$)

Production Example 52

(E)-3-(4-oxo-7-trifluoromethyl-4,5-dihydropyrrolo[1,2-a]-quinoxalin-1-yl)acrylic acid (Compound No.: C-10)

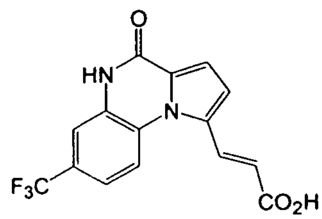

$^1$H-NMR(DMSO-$d_6$,δ):6.55(1H,d,J=15.4Hz),7.1-7.3(2H,m), 7.6-7.7(2H,m),7.95(1H,d,J=9.6Hz),8.02(1H,d,J=15.4Hz), 11.66(1H,br s),12.65(1H,br s)
MS(m/z):322($M^+$), 277(base)

Example 1: Formulation Example of Tablets

| | mg/tablet |
|---|---|
| Active ingredient | 5.0 |
| Starch | 10.0 |
| Lactose | 73.0 |
| Carboxymethyl cellulose calcium | 10.0 |
| Talc | 1.0 |
| Magnesium stearate | 1.0 |
| | 100.0 |

The active ingredient was pulverized to the particle size not greater than 70 μm, to which starch, lactose and carboxymethyl cellulose calcium were added and mixed thoroughly. Ten (10)% starch paste was added to the above powdery mixture, mixed and stirred to form granules. After drying, their particle size was dressed to around 1000 μm, with which talc and magnesium stearate were mixed and tabletted.

## Claims

1. A treating agent of overactive bladder syndrome, pollakiuria, urinary incontinence, dysuria in benign prostatic hyperplasia or urolithiasis, which is **characterized by** comprising a compound having phosphodiesterase type 9 (PDE9)-inhibiting activity as the active ingredient.

2. A treating agent according to Claim 1, in which the compound having PDE9-inhibiting activity is selected from thienopyrimidine derivatives represented by Formula (I),

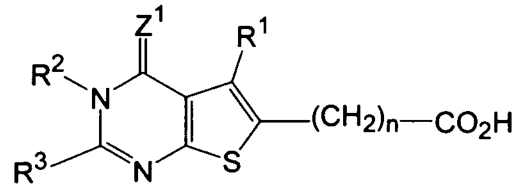

(I)

in the formula,

$R^1$ stands for hydrogen, $C_{1-6}$ alkyl, $C_{1-6}$ alkoxy$C_{1-6}$ alkyl, or $C_{1-6}$ haloalkyl having 1-9 halogen atoms,
$R^2$ stands for hydrogen, $C_{1-6}$ alkyl, phenyl$C_{1-6}$ alkyl or amino,
$R^3$ either stands for $C_{2-6}$ alkyl, $C_{2-6}$ alkenyl, carbamoyl$C_{1-6}$ alkyl, amino$C_{1-6}$ alkyl, $C_{1-6}$ alkylamino$C_{1-6}$ alkyl, di-($C_{1-6}$ alkyl) amino$C_{1-6}$ alkyl, $C_{1-6}$ alkylthio or Y-X-, or
$R^2$ and $R^3$ may together form tetramethylene,
X stands for a direct bond or $CH_2$, CH(OH), $CH(C_6H_5)$, CO, $CH_2CH_2$, $CH_2CO$, $COCH_2$, S, O, or NH,
Y stands for an aromatic carbocyclic group, aromatic heterocyclic group, $C_{4-7}$ cycloalkyl, $C_{4-7}$ cycloalkenyl, 5- to 7-membered saturated heterocyclic group containing 1 or 2 nitrogen atoms, or 5- to 7-membered saturated heterocyclic group forming a condensed ring with a 5- to 6-membered saturated cyclic group and containing 1 or 2 nitrogen atoms, each of which may be optionally substituted with 1-3 substituents selected from halogen, $C_{1-6}$ alkyl, $C_{1-6}$ haloalkyl having 1-9 halogen atoms, $C_{1-6}$ alkoxy, $C_{1-6}$ haloalkoxy having 1-9 halogen atoms, $C_{1-6}$ alkylthio, $C_{1-6}$ haloalkylthio having 1-9 halogen atoms, $C_{1-4}$ alkylenedioxy, carboxy, $C_{1-6}$ alkoxycarbonyl, oxo, amino, nitro and phenyl,
$Z^1$ stands for S or O, and
n is 0 or an integer of 1-4,
and salts thereof,
quinazoline derivatives represented by Formula (II),

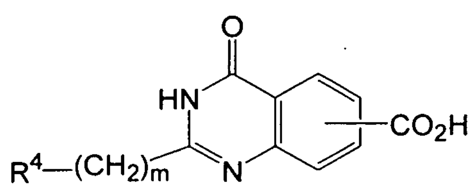

(II)

in the formula,
$R^4$ stands for phenyl or aromatic heterocyclic group which may be optionally substituted with 1-3 substituents selected from halogen, $C_{1-6}$ alkyl, $C_{1-6}$ haloalkyl having 1-9 halogen atoms and $C_{1-6}$ alkoxy, and
m is an integer of 1-3,
and salts thereof.
quinoxaline derivatives represented by Formula (III),

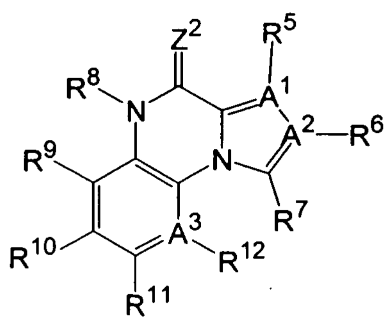

(III)

in the formula,

$R^5$ and $R^6$ each independently stands for hydrogen; halogen; $C_{1-6}$ alkyl or $C_{1-6}$ alkoxy each of which is optionally substituted with hydroxy, halogen, $C_{1-6}$ alkoxy, $C_{1-6}$ haloalkoxy having 1 - 9 halogen atoms, carboxy, $C_{1-6}$ alkoxycarbonyl, $C_{1-6}$ alkanoyl, amino, amido, carbamoyl, oxo, carbocyclic group or heterocyclic group; acyl which is optionally substituted with hydroxy, $C_{1-6}$ alkyl, $C_{2-6}$ alkenyl, $C_{2-6}$ alkynyl, $C_{1-6}$ haloalkyl having 1 - 9 halogen atoms, $C_{1-6}$ alkoxy, $C_{1-6}$ haloalkoxy having 1 - 9 halogen atoms, amino, carbocyclic group or heterocyclic group; amino which is optionally substituted with 1 - 2 substituents selected from $C_{1-6}$ alkyl, $C_{2-6}$ alkenyl, $C_{2-6}$ alkynyl, alkanoyl, carbocyclic group and heterocyclic group; hydroxy; or pyrimidinyl which is optionally substituted with halogen, $C_{1-6}$ alkyl, $C_{1-6}$ alkoxy, $C_{1-6}$ haloalkoxy having 1 - 9 halogen atoms, nitro or amino,

$R^7$ stands for $C_{1-9}$ alkyl, $C_{2-9}$ alkenyl or $C_{2-9}$ alkynyl which are optionally substituted with hydroxy, halogen, $C_{1-6}$ alkoxy, $C_{1-6}$ haloalkoxy having 1 - 9 halogen atoms, carboxy, $C_{1-6}$ alkoxycarbonyl, alkanoyl, amino (here the amino group may further be substituted with 1 - 2 substituents selected from $C_{1-6}$ alkyl, $C_{2-6}$ alkenyl, $C_{2-6}$ alkynyl, $C_{1-6}$ haloalkyl having 1 - 9 halogen atoms, alkanoyl, carbocyclic group and heterocyclic group), amido, carbamoyl, oxo, carbocyclic or heterocyclic group (here the carbocyclic group and heterocyclic group each may further be substituted with hydroxy, halogen, $C_{1-6}$ alkyl, $C_{2-6}$ alkenyl, $C_{2-6}$ alkynyl, $C_{1-6}$ alkoxy, carboxy, $C_{1-6}$ alkoxycarbonyl, amino, amido or carbamoyl); aryl, saturated carbocyclic group or saturated heterocyclic group, each of which is optionally substituted with halogen, hydroxy, $C_{1-6}$ alkyl, $C_{2-6}$ alkenyl, $C_{2-6}$ alkynyl, $C_{1-6}$ alkoxy (here the $C_{1-6}$ alkyl, $C_{2-6}$ alkenyl, $C_{2-6}$ alkynyl and $C_{1-6}$ alkoxy may further be substituted with halogen, hydroxy, $C_{1-6}$ alkoxy, $C_{1-6}$ haloalkoxy having 1 - 9 halogen atoms, carboxy, $C_{1-6}$ alkoxycarbonyl, alkanoyl, amino, amido, carbamoyl, carbocyclic group or heterocyclic group, independently of each other), $C_{1-6}$ haloalkoxy having 1 - 9 halogen atoms, carboxy, $C_{1-6}$ alkoxycarbonyl, alkanoyl, amino, amido, carbamoyl, carbocyclic group or heterocyclic group; carboxy; $C_{1-6}$ alkoxycarbonyl (here the $C_{1-6}$ alkoxy moiety in the $C_{1-6}$ alkoxycarbonyl may further be substituted with hydroxy, halogen, $C_{1-6}$ alkoxy, $C_{1-6}$ haloalkoxy having 1 - 9 halogen atoms, carboxy, $C_{1-6}$ alkoxycarbonyl, amino, amido, carbamoyl, carbocyclic group or heterocyclic group); amido (here the amino moiety in the amido may further be substituted with 1 - 2 substituents selected from $C_{1-6}$ alkyl, $C_{2-6}$ alkenyl, $C_{2-6}$ alkynyl, $C_{1-6}$ haloalkyl having 1 - 9 halogen atoms, alkanoyl, carbocyclic group and heterocyclic group) or carbamoyl (here the amino moiety in the carbamoyl may further be substituted with 1 - 2 substituents selected from $C_{1-6}$ alkyl, $C_{2-6}$ alkenyl, $C_{2-6}$ alkynyl, $C_{1-6}$ haloalkyl having 1 - 9 halogen atoms, alkanoyl, carbocyclic group and heterocyclic group),

$R^8$ stands for hydrogen; hydroxy; $C_{1-6}$ alkyl which is optionally substituted with hydroxy, halogen, $C_{1-6}$ alkoxy, $C_{1-6}$ haloalkoxy having 1 - 9 halogen atoms, carboxy, $C_{1-6}$ alkoxycarbonyl, alkanoyl, amino, amido, carbamoyl or oxo; or amino which is optionally substituted with 1 - 2 substituents selected from $C_{1-6}$ alkyl, $C_{2-6}$ alkenyl, $C_{2-6}$ alkynyl, $C_{1-6}$ haloalkyl having 1 - 9 halogen atoms, alkanoyl, carbocyclic group and heterocyclic group,

$R^9$ and $R^{12}$ each independently stands for hydrogen; halogen; $C_{1-6}$ alkyl, $C_{2-6}$ alkenyl or $C_{1-6}$ alkoxy each of which is optionally substituted with hydroxy, halogen, $C_{1-6}$ alkoxy, $C_{1-6}$ haloalkoxy having 1 - 9 halogen atoms, carboxy, $C_{1-6}$ alkoxycarbonyl, alkanoyl, amino, amido, carbamoyl or oxo; cyano; or nitro,

$R^{10}$ and $R^{11}$ each independently stands for hydrogen; halogen; $C_{1-6}$ alkyl, $C_{2-6}$ alkenyl, $C_{2-6}$ alkynyl or $C_{1-6}$ alkoxy each of which is optionally substituted with hydroxy, halogen, $C_{1-6}$ alkoxy, $C_{1-6}$ haloalkoxy having 1 - 9 halogen atoms, carboxy, $C_{1-6}$ alkoxycarbonyl, alkanoyl, amino (here the amino may further be substituted with 1 - 2 substituents selected from $C_{1-6}$ alkyl, $C_{2-6}$ alkenyl, $C_{2-6}$ alkynyl, $C_{1-6}$ haloalkyl having 1 - 9 halogen atoms, alkanoyl, carbocyclic group and heterocyclic group), amido, carbamoyl, oxo, carbocyclic group or heterocyclic group (here the carbocyclic group and heterocyclic group each may further be substituted with hydroxy, halogen, $C_{1-6}$ alkyl, $C_{2-6}$ alkenyl, $C_{2-6}$ alkynyl, $C_{1-6}$ alkoxy, carboxy, $C_{1-6}$ alkoxycarbonyl, amino, amido or carbamoyl cyano, amino which is optionally substituted with 1 - 2 substituents selected from $C_{1-6}$ alkyl, $C_{2-6}$ alkenyl, $C_{2-6}$ alkynyl (here the $C_{1-6}$ alkyl, $C_{2-6}$alkenyl and $C_{2-6}$ alkynyl may further be substituted with, independently of each other, hydroxy, halogen, $C_{1-6}$ alkoxy, $C_{1-6}$ haloalkoxy having 1 - 9 halogen atoms, carboxy, $C_{1-6}$ alkoxycarbonyl, $C_{1-6}$ alkanoyl, amino, amido, carbamoyl, oxo, carbocyclic group and heterocyclic group), alkanoyl, carbocyclic group and heterocyclic group (here the carbocyclic group and heterocyclic group each may further be substituted with hydroxy, halogen, $C_{1-6}$ alkyl, $C_{2-6}$ alkenyl, $C_{2-6}$ alkynyl, $C_{1-6}$ alkoxy, carboxy, $C_{1-6}$ alkoxycarbonyl, amino, amido or carbamoyl); carbocyclic group or heterocyclic group each of which is optionally substituted with hydroxy, halogen, $C_{1-6}$ alkyl, $C_{2-6}$ alkenyl, $C_{2-6}$ alkynyl, $C_{1-6}$ alkoxy (here the $C_{1-6}$ alkyl, $C_{2-6}$ alkenyl, $C_{2-6}$ alkynyl and $C_{1-6}$ alkoxy may further be substituted with, independently of each other, hydroxy, halogen, $C_{1-6}$ alkoxy, $C_{1-6}$ haloalkoxy having 1 - 9 halogen atoms, carboxy, $C_{1-6}$ alkoxycarbonyl, $C_{1-6}$ alkanoyl, amino, amido, carbamoyl, oxo, carbocyclic group or heterocyclic group), $C_{1-6}$ haloalkoxy having 1 - 9 halogen atoms, carboxy, $C_{1-6}$ alkoxycarbonyl, amino, amido or carbamoyl; $COR^{13}$; or $SO_2R^{13}$,

$R^{13}$ stands for hydrogen; hydroxy; $C_{1-6}$ alkyl, $C_{2-6}$ alkenyl, $C_{2-6}$ alkynyl or $C_{1-6}$ alkoxy, each of which is optionally substituted with hydroxy, halogen, $C_{1-6}$ alkoxy, $C_{1-6}$ haloalkoxy having 1 - 9 halogen atoms, carboxy, $C_{1-6}$ alkoxycarbonyl, alkanoyl, amino (here the amino may further be substituted with 1 - 2 substituents selected

from $C_{1-6}$ alkyl, $C_{2-6}$ alkenyl, $C_{2-6}$ alkynyl, $C_{1-6}$ haloalkyl having 1 - 9 halogen atoms, alkanoyl, carbocyclic group and heterocyclic group), amido, oxo, carbocyclic group or heterocyclic group (here the carbocyclic group and heterocyclic group each may further be substituted with hydroxy, halogen, $C_{1-6}$ alkyl, $C_{2-6}$ alkenyl, $C_{2-6}$ alkynyl, $C_{1-6}$ alkoxy, carboxy, $C_{1-6}$ alkoxycarbonyl, amino, amido or carbamoyl); amino which may be substituted with 1 - 2 substituents selected from $C_{1-6}$ alkyl, $C_{2-6}$ alkenyl, $C_{2-6}$ alkynyl, (here the $C_{1-6}$ alkyl, $C_{2-6}$ alkenyl and $C_{2-6}$ alkynyl may further be substituted with, independently of each other, hydroxy, halogen, $C_{1-6}$ alkoxy, $C_{1-6}$ haloalkoxy having 1 - 9 halogen atoms, carboxy, $C_{1-6}$ alkoxycarbonyl, alkanoyl, amino, amido, carbamoyl, oxo, carbocyclic group or heterocyclic group), $C_{1-6}$ haloalkyl having 1 - 9 halogen atoms, alkanoyl, carbocyclic group and heterocyclic group (here the carbocyclic group and heterocyclic group each may further be substituted with hydroxy, halogen, $C_{1-6}$ alkyl, $C_{2-6}$ alkenyl, $C_{2-6}$ alkynyl, $C_{1-6}$ alkoxy, carboxy, $C_{1-6}$ alkoxycarbonyl, amino, amido or carbamoyl); or aziridin-1-yl, azetidin-1-yl, pyrrolidin-1-yl, piperidin-1-yl, piperazin-1-yl, morpholin-1-yl or pyrazol-1-yl, each of which may be substituted with 1-2 substituents selected from hydroxy, halogen, $C_{1-6}$ alkyl, $C_{2-6}$ alkenyl, $C_{2-6}$ alkynyl, $C_{1-6}$ alkoxy (here the $C_{1-6}$ alkyl, $C_{2-6}$ alkenyl, $C_{2-6}$ alkynyl and $C_{1-6}$ alkoxy may further be substituted with, independently of each other, hydroxy, halogen, $C_{1-6}$ alkoxy, $C_{1-6}$ haloalkoxy having 1 - 9 halogen atoms, carboxy, $C_{1-6}$ alkoxycarbonyl, alkanoyl, amino, amido, carbamoyl, oxo, carbocyclic group or heterocyclic group), $C_{1-6}$ haloalkoxy having 1 - 9 halogen atoms, carboxy, $C_{1-6}$ alkoxycarbonyl, alkanoyl, amino (here the amino may further be substituted with 1 - 2 substituents selected from $C_{1-6}$ alkyl, $C_{2-6}$ alkenyl, $C_{2-6}$ alkynyl, $C_{1-6}$ haloalkyl having 1 - 9 halogen atoms, alkanoyl, carbocyclic group and heterocyclic group), amido, carbamoyl, oxo, carbocyclic group and heterocyclic group (here the carbocyclic group and heterocyclic group each may further be substituted with hydroxy, halogen, $C_{1-6}$ alkyl, $C_{2-6}$ alkenyl, $C_{2-6}$ alkynyl, $C_{1-6}$ alkoxy, carboxy, $C_{1-6}$ alkoxycarbonyl, amino, amido or carbamoyl),

$Z^2$ stands for S or O,

$A^1$, $A^2$ and $A^3$ stand for N or C, independently of each other, with the proviso that $R^5$, $R^6$ and $R^{12}$ are respectively absent where $A^1$, $A^2$ and $A^3$ respectively stand for N,

and salts thereof;

pyrazolopyrimidine derivatives represented by Formula (IV),

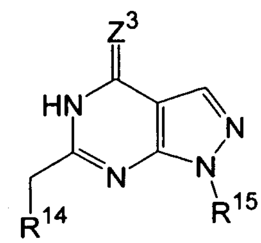

(IV)

in the formula,

$R^{14}$ stands for phenyl which is substituted with 1-5 substituents selected from halogen, $C_{1-6}$ alkyl, trifluoromethyl, trifluoromethoxy, cyano, hydroxy, nitro and $C_{1-6}$ alkoxy,

$R^{15}$ stands for pentan-3-yl or $C_{4-6}$ cycloalkyl, and

$Z^3$ stands for S or O,

and salts thereof; and

pyrazolopyrimidine derivatives represented by Formula (V)

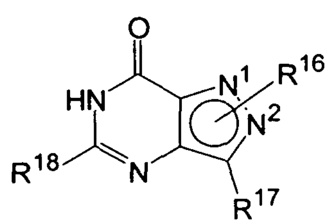

(V)

in the formula,

$R^{16}$ stands for hydrogen or $C_{1-6}$ alkyl, here the $R^{16}$ binding to $N^1$ or $N_2$,

$R^{17}$ stands for $C_{1-6}$ alkyl which is optionally substituted with hydroxy or alkoxy; $C_{3-7}$ cycloalkyl which is optionally substituted with alkyl, hydroxy or alkoxy; saturated 5- to 6-membered heterocyclic ring which is optionally substituted with alkyl, hydroxy or alkoxy; $het^1$; or $Ar^1$,

$R^{18}$ stands for $C_{1-6}$ alkyl which is optionally substituted with 1-2 substituents selected from optionally $Ar^2$-substituted or $C_{1-6}$ alkyl-substituted $C_{3-7}$ cycloalkyl, $OAr^2$, $SAr^2$, $NHC(O)C_{1-6}$ alkyl, $het^2$, xanthine and naphthalene, here $Ar^1$ and $Ar^2$ standing for the group represented by the following formula (VI), independently of each other,

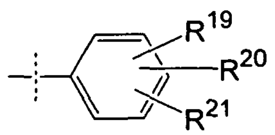 (VI)

[in the formula, $R^{19}$, $R^{20}$ and $R^{21}$ are either selected from hydrogen, halogen, phenoxy, phenyl, $CF_3$, $OCF_3$, $R^{22}$, $SR^{22}$ and $OR^{22}$ (here $R^{22}$ standing for $het^3$ or $C_{1-6}$ alkyl which is optionally substituted with phenyl, which phenyl being optionally further substituted with 1-3 substituents selected from halogen, $CF_3$, $OCF_3$, $C_{1-6}$ alkyl and $C_{1-6}$ alkoxy), or $R^{19}$ and $R^{20}$ together forming 3- or 4-atomic linker optionally containing 1-2 hetero atoms selected from O, S and N],

here $het^1$, $het^2$ and $het^3$ may be the same or different, standing for aromatic 5- to 6-membered heterocyclic ring containing 1-3 hetero atoms selected from O, S and N, the heterocyclic ring being optionally substituted with 1-3 substituents selected from $C_{1-6}$ alkyl, $C_{1-6}$ alkoxy, halogen, and phenyl which may further be substituted with 1-3 substituents selected from halogen and $C_{1-6}$ alkyl,

and salts thereof.

**3.** Pharmaceutical compositions for treatment of overactive bladder syndrome, pollakiuria, urinary incontinence, dysuria in benign prostatic hyperplasia or urolithiasis, which comprise compounds having PDE9-inhibiting activity, together with non-toxic excipients.

**4.** A method for treating overactive bladder syndrome, pollakiuria, urinary incontinence, dysuria in benign prostatic hyperplasia or urolithiasis, which is **characterized by** administering an effective amount of a compound having PDE9-inhibiting activity to a patient in need of the treatment.

**5.** Use of a comound having PDE9-inhibiting activity for treatment of overactive bladder syndrome, pollakiuria, urinary incontinence, dysuria in benign prostatic hyperplasia or urolithiasis.

**INTERNATIONAL SEARCH REPORT**

International application No.
PCT/JP2007/074361

A. CLASSIFICATION OF SUBJECT MATTER
*A61K45/00*(2006.01)i, *A61K31/4985*(2006.01)i, *A61K31/519*(2006.01)i, *A61P13/00*(2006.01)i, *A61P13/02*(2006.01)i, *A61P13/04*(2006.01)i, *A61P43/00*(2006.01)i, *C07D487/04*(2006.01)i, *C07D495/04*(2006.01)i

According to International Patent Classification (IPC) or to both national classification and IPC

B. FIELDS SEARCHED

Minimum documentation searched (classification system followed by classification symbols)
A61K45/00, A61K31/00-31/80, C07D487/04, C07D495/04

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2008 |
| Kokai Jitsuyo Shinan Koho | 1971-2008 | Toroku Jitsuyo Shinan Koho | 1994-2008 |

Electronic data base consulted during the international search (name of data base and, where practicable, search terms used)
BIOSIS(STN), CAplus(STN), EMBASE(STN), MEDLINE(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| P,X | WO 2006/135080 A1 (ASKA PHARM CO., LTD.), 21 December, 2006 (21.12.06), Full text (Family: none) | 1-3 |
| E,X | WO 2008/018306 A1 (ASKA PHARM CO., LTD.), 14 February, 2008 (14.02.08), Full text (Family: none) | 1-3 |
| X<br>Y | Wheeler et al., 'Regulation of cyclic nucleotides in the urinary tract', J. Smooth Muscle Res. (2005), 41(1):1-21 | 1,3<br>2 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>25 March, 2008 (25.03.08) | Date of mailing of the international search report<br>08 April, 2008 (08.04.08) |
| Name and mailing address of the ISA/<br>Japanese Patent Office<br>Facsimile No. | Authorized officer<br><br>Telephone No. |

Form PCT/ISA/210 (second sheet) (April 2007)

**INTERNATIONAL SEARCH REPORT**

International application No.
PCT/JP2007/074361

C (Continuation). DOCUMENTS CONSIDERED TO BE RELEVANT

| Category* | Citation of document, with indication, where appropriate, of the relevant passages | Relevant to claim No. |
|---|---|---|
| Y | WO 2004/053495 A1 (BAYER HEALTHCARE AG.), 24 June, 2004 (24.06.04), Full text & AU 2003283427 A1 & EP 1573331 A1 | 1-3 |
| Y | WO 2004/053494 A1 (BAYER HEALTHCARE AG.), 24 June, 2004 (24.06.04), Full text & AU 2003294728 A1 & EP 1573333 A1 | 1-3 |
| Y | WO 2004/053492 A1 (BAYER HEALTHCARE AG.), 24 June, 2004 (24.06.04), Full text & AU 2003288171 A1 & EP 1573332 A1 | 1-3 |
| Y | WO 2004/053493 A1 (BAYER HEALTHCARE AG.), 24 June, 2004 (24.06.04), Full text & AU 2003289894 A1 & EP 1576372 A1 | 1-3 |
| Y | JP 2005-508978 A (PFIZER INC.), 07 April, 2005 (07.04.05), Full text & WO 2003/037432 A1 & US 2004/023989 A1 & EP 1444009 A1 & AU 2002328122 A1 & KR 2004053210 A & BR 200213817 A & HU 200401998 A2 & US 2005/070557 A1 & CN 1575191 A & ZA 200402173 A & MX 2004004170 A1 | 2 |
| Y | JP 2005-511575 A (PFIZER INC.), 28 April, 2005 (28.04.05), Full text & WO 2003/037899 A1 & US 2003/195205 A1 & EP 1440073 A1 & AU 2002339572 A1 & BR 200214096 A & TW 200300081 A & MX 2004004171 A1 | 2 |
| Y | JP 2006-507242 A (BAYER AG.), 02 March, 2006 (02.03.06), Full text & WO 2004/018474 A1 & DE 10238723 A1 & AU 2003258601 A1 & EP 1534711 A1 & US 2006/106035 A1 | 2 |

Form PCT/ISA/210 (continuation of second sheet) (April 2007)

**INTERNATIONAL SEARCH REPORT**

International application No.
PCT/JP2007/074361

**Box No. II Observations where certain claims were found unsearchable (Continuation of item 2 of first sheet)**

This international search report has not been established in respect of certain claims under Article 17(2)(a) for the following reasons:

1. ☒ Claims Nos.: 4-5
because they relate to subject matter not required to be searched by this Authority, namely:
Claims 4 to 5 pertain to methods for treatment of the human body by therapy and thus relate to a subject matter which this International Searching Authority is not required, under the provisions of the PCT Rule 39.1(iv), to search.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.
2. ☐ As all searchable claims could be searched without effort justifying additional fees, this Authority did not invite payment of additional fees.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** the

☐ The additional search fees were accompanied by the applicant's protest and, where applicable, payment of a protest fee.

☐ The additional search fees were accompanied by the applicant's protest but the applicable protest fee was not paid within the time limit specified in the invitation.

Form PCT/ISA/210 (continuation of first sheet (2)) (April 2007)

INTERNATIONAL SEARCH REPORT

International application No. PCT/JP2007/074361

<Subject of search>

Claims 1 and 3 relate to "a therapeutic agent for difficulty in urination associated with overactive bladder, frequent urination, urinary incontinence or prostatomegaly or urinary calculus" which comprises a compound defined by a desired property "a compound having a PDE9-inhibiting activity" as an active ingredient. Claims 1 and 3 include all of the compounds having the properties. However, among these compounds, those which are disclosed in the meaning within PCT Article 5 are limited to several compounds represented by the general formulae (I) to (V). Therefore, it does not appear that these claims are supported by the disclosure of the description in the meaning within PT Article 6.

Further, even though the common technical knowledge at the time of filing the present application is taken into the consideration, it appears that it is impossible to specify the scope of the "compound having a PDE9-inhibiting activity". Thus, claims 1 and 3 do not comply with the requirement of clearness under PCT Article 6, too.

Such being the case, the search was made on the relationship between "a PDE9-inhibiting activity" and "a therapeutic agent for difficulty in urination associated with overactive bladder, frequent urination, urinary incontinence or prostatomegaly or urinary calculus", and also made on "a therapeutic agent for difficulty in urination associated with overactive bladder, frequent urination, urinary incontinence or prostatomegaly or urinary calculus" comprising, as an active ingredient, any one of the compounds represented by the general formulae (I) to (V) which are specifically set forth in the description.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 03037432 A
- WO 03037899 A
- WO 2004018474 A


### Non-patent literature cited in the description


- *J. Biol. Chemistry,* 1998, vol. 273 (25), 15559-15564
- *SYNTHESIS,* 1980, 150-151
- *Bioorg. Med. Chem. Lett.,* 2002, 1275-1278